# EUROPEAN PATENT SPECIFICATION

(11) **EP 3 000 888 B1**
(45) Date of publication and mention of the grant of the patent: **05.12.2018**
(21) Application number: 14186796.0
(22) Date of filing: 29.09.2014
(51) Int. Cl.: C12P 7/24

(54) **Process for converting ferulic acid into vanillin**
Verfahren zur Umwandlung von Ferulasäure in Vanillin
Procedée pour la conversion d'acide ferulique en vanilline

(43) Date of publication of application: 30.03.2016
(73) Proprietor: Symrise AG, 37603 Holzminden (DE)
(72) Inventor: Fleige, Christian, 59075 Hamm (DE); Steinbüchel, Alexander, 48341 Altenberge (DE); Pesaro, Manuel, 37603 Holzminden (DE); Meyer, Florian, 48161 Münster-Roxel (DE)
(74) Representative: Fabry, Bernd

(56) References cited:
- WO-A1-2012/172108
- C. FLEIGE ET AL: "Investigation of the Amycolatopsis sp. Strain ATCC 39116 Vanillin Dehydrogenase and Its Impact on the Biotechnical Production of Vanillin", APPLIED AND ENVIRONMENTAL MICROBIOLOGY, vol. 79, no. 1, 12 October 2012 (2012-10-12), pages 81-90, XP055106545, ISSN: 0099-2240, DOI: 10.1128/AEM.02358-12
- DIANA DI GIOIA ET AL: "Metabolic engineering of Pseudomonas fluorescens for the production of vanillin from ferulic acid", JOURNAL OF BIOTECHNOLOGY, ELSEVIER SCIENCE PUBLISHERS, AMSTERDAM, NL, [Online] vol. 156, no. 4, 20 December 2010 (2010-12-20), pages 309-316, XP002684679, ISSN: 0168-1656, DOI: 10.1016/J.JBIOTEC.2011.08.014 [retrieved on 2011-08-22]
- GRAF NADJA ET AL: "Genetic engineering ofPseudomonas putidaKT2440 for rapid and high-yield production of vanillin from ferulic acid", APPLIED MICROBIOLOGY AND BIOTECHNOLOGY, SPRINGER, DE, vol. 98, no. 1, 18 October 2013 (2013-10-18), pages 137-149, XP035329011, ISSN: 0175-7598, DOI: 10.1007/S00253-013-5303-1 [retrieved on 2013-10-18]
- CHRISTIAN FLEIGE ET AL: "Construction of expression vectors for metabolic engineering of the vanillin-producing actinomycete Amycolatopsis sp. ATCC 39116", APPLIED MICROBIOLOGY AND BIOTECHNOLOGY, vol. 98, no. 14, 18 April 2014 (2014-04-18), pages 6387-6395, XP055148582, ISSN: 0175-7598, DOI: 10.1007/s00253-014-5724-5
- ACHTERHOLT ET AL: "Identification of Amycolatopsis sp. strain HR167 genes, involve in the bioconversion of ferulic acid to vanillin", APPLIED MICROBIOLOGY AND BIOTECHNOLOGY, SPRINGER, DE, vol. 54, no. 6, 1 January 2000 (2000-01-01), pages 799-807, XP002263786, ISSN: 0175-7598, DOI: 10.1007/S002530000431
- BALJINDER KAUR ET AL: "Biotechnological and Molecular Approaches for Vanillin Production: a Review", APPLIED BIOCHEMISTRY AND BIOTECHNOLOGY, vol. 169, no. 4, 1 February 2013 (2013-02-01), pages 1353-1372, XP055064352, ISSN: 0273-2289, DOI: 10.1007/s12010-012-0066-1

## Description

### FIELD OF INVENTION

The present invention belongs to the area of biotechnology and refers to an improved fermentation process for converting ferulic acid into vanillin by means of genetically engineered microorganisms.

### STATE OF THE ART

With an annual worldwide demand of more than 16,000 tons, vanillin is one of the most important flavour compounds. It is widely used in various applications, such as the production of fragrances, pharmaceutics, foods and beverages. The vast majority (99%) of the industrially used vanillin is chemically synthesized from lignin and crude oil derivatives, since the extraction of vanillin from the pods of *Vanilla planifolia* is highly cost and labour intensive. Although chemically synthesized vanillin can be produced at a very attractive price, there is a growing interest in the production of natural vanillin, originated from biotechnical approaches. Due to the customers increasing concerns for 'natural', 'healthy' and 'bio' products, natural flavours have a higher economic value in comparison to the chemically generated versions. This has led to extensive research to produce valuable compounds in environmentally friendly processes based from renewable substrates.

In addition to the use as a flavouring agent, vanillin and other structurally related phenolic compounds exhibit antimicrobial and antioxidant properties and are therefore also used in various pharmaceutical applications and as preservatives. Especially such bioactive functions are dependent on the correct stereochemistry. Biocatalysis is highly stereo-, regio-, and chemospecific, while synthetic production may lead to racemic mixtures and unintended by-products. Furthermore, chemical approaches often face the problem of environmentally problematic reaction conditions and the production of hazardous waste materials.

The actinomycete *Amycolatopsis* sp. ATCC 39116 has an outstanding tolerance towards vanillin in fermentation processes and is therefore used by the industry for the production of natural vanillin from ferulic acid and other structurally related compounds. Ferulic acid is the most abundant hydroxycinnamic acid in the plant world, as it is an important component of the cell wall of higher plants. In this case vanillin can be labelled as 'natural flavour' since it is extracted from natural sources or "obtained by appropriate physical, enzymatic or microbiological processes from material of vegetable, animal or microbiological origin".

Unfortunately, microbial produced vanillin is often rapidly converted due to detoxification or catabolized within the organism's metabolism to serve as source of carbon and energy. Thus, much research was done to establish vanillin production pathways in model organisms like *E. coli* or *S*. *cerevisiae* that are not able to degrade vanillin. However, these approaches did not lead to an industrial application, because of the bacteria's insufficient tolerance against the product.

To render the microbial production economically attractive, first attempts were made to engineer the metabolism of *Amycolatopsis* sp. ATCC 39116 towards an efficient accumulation of vanillin [Fleige et al. "Investigation of the Amyolatopsis sp. Strain ATCC 39116 vanillin dehydrogenase and its impact on the biotechnical production of vanillin", Appl. Environ. Microbiol. 79, p 81-90 (2013**)].** The document relates also to the mutant ATCC 39116 Δvdh::Km^{r} which differs from the wild form in that one gene has been replaced by kanamycin resistance cassette. The mutant is reported to be more efficient with regard to vanillin production as the wild form.

The catabolism of ferulic acid in *Amycolatopsis* sp. ATCC 39116, as well as in many vanillin-producing strains, proceeds via a consistent coenzyme A-dependent, non β-oxidative pathway with vanillin as the result of two enzymatic steps. These two reactions are catalyzed by the enzymes feruloyl-CoA synthetase (Fcs) and enoyl-CoA hydratase/aldolase (Ech), respectively (2, 6, 7, 14-17). Vanillin is then further converted to vanillic acid by the enzyme vanillin dehydrogenase (Vdh). Deletion of the encoding gene (*vdh*) in *Amycolatopsis* sp. ATCC 39116 led to a strongly enhanced accumulation of vanillin and a reduction of more than 90% of its catabolism in biotransformation experiments.

To gain further access to an efficient genetic engineering of *Amycolatopsis* sp. ATCC 39116, new molecular tools were developed [Fleige et al. "Construction of expression vectors for metabolic engineering of the vanillin-producing actinomycete Amycolatopsis sp. Strain HR167 genes ...", Appl. Microbiol. Biotechnol. 10. 1007/s00253-014-5724-5 (2014**)].** The use of suicide plasmids und expression vectors now facilitates genetic modifications of the genome of this promising production strain.

Therefore, the problem underlying the present invention has been to develop an improved fermentation process that allows converting ferulic acid into vanillin with improved yields. More particularly the invention relates to improvements in the metabolisms of microorganisms of the *Amycolatopsis* species in order to engineer an efficient production strain for natural vanillin.

### DESCRIPTION OF THE INVENTION

A first object of the present invention is a culture of Amycolatopsis sp. ATCC 39116 - mutant *Amycolatopsis* sp. ATCC 39116 Δ*vdh* which is F33 or mutant *Amycolatopsis* sp. ATCC 39116 Δ*vdh::permE*::echfcs* which is F84 and converts ferulic acid into vanillin, wherein the mutant F33 is obtained by
(a1) digesting *E. coli* - Amycolatopsis shuttle vector p6apra with *Nae*I/*SacI*I to eliminate the essential part of the origin of replication for Amycolatopsis and to obtain the resulting fragment (3575 bp) containing *oriV* for replication in *E. coli,* the apramycin resistance gene and several unique restriction sites for cloning;
(a2) religating the fragment and transforming *E. coli* with the resulting plasmid p6sui;
(a3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(a4) deleting of the vdh gene by homologous recombination, wherein the flanking regions upstream and downstream of *vdh* in the genome of *Amycolatopsis* sp. ATCC 39116 are amplified using the following oligonucleotides:
   upstream region:
      *vdh*LF*_*for3 (5'-TCGTACTTCGCGGTGATCTCGTGG-3')
         and
      *vdh*LF_rev_RF (5'-GCGCATCAGGAGTGACACGCGCGGCGGGGGCG-3');
   downstream region:
      *vdh*RF_for_LF (5'-CGCCCCCGCCGCGCGTGTCACTCCTGATGCGC-3') and
      *vdh*RF_rev3 (5'-TTGCAGTCCTTTGTAGAGCGACACG-3');
(a5) purifiying the resulting amplificates and combining them in a subsequent fusion-PCR using primers *vdh*LF for3 and *vdh*RF_rev3;
(a6) cloning the resulting fragment Δ*vdh* including the upstream and downstream regions of *vdh* into the *Eco*RV-site of the suicide plasmid p6suigusA;
(a7) isolating the resulting plasmid p6suigusA::Δ*vdh* from *E. coli* and transferring the vector to *Amycolatopsis* sp. ATCC 39116; and
(a8) selecting transformants of *Amycolatopsis* sp. ATCC 39116 with regard to correct genotype and cultivating them to obtain a second recombinant event;
   and the mutant F84 is obtained by
(b1) digesting *E. coli* - *Amycolatopsis* shuttle vector p6apra with *Nae*I/*Sac*II to eliminate the essential part of the origin of replication for Amycolatopsis and to obtain the resulting fragment (3575 bp) containing *oriV* for replication in *E. coli,* the apramycin resistance gene and several unique restriction sites for cloning;
(b2) religating the fragment and transforming *E. coli* with the resulting plasmid p6sui;
(b3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(b4) amplifying the genes ech and fcs together from genomic DNA of *Amycolatopsis* sp. ATCC 39116 using oligonucleotides
   *ech_*for*_Sac*I (5'-AAAAGAGCTCTAAGGAGGTGACAACTGCTGGC CGCGCTCG-3')
      and
   *fcs*_rev_*X*baI (5'-AAAATCTAGACCACAGAGTAGCCGCAGCGGG-3');
(b5) digesting the PCR product with Sacl and Xbal and subsequently cloning it into the *Sac*I/*Xba*I-linearized pBluescript SK⁻.
(b6) transferring the resulting plasmid *pSK::echfcs* to *E. coli* Mach-1 T;
(b7) digesting *pSK::echfcs* with *Eco*ICRI and *Hin*dIII*,* and ligating the resulting fragment, containing both genes, with the *Fsp*AI/HindIII-linearized expression vector p6permE, collinear to *permE*;*
(b8) amplifying a fragment, comprising the promoter *permE** and the genes *ech* and *fcs* from the resulting plasmid p6permE::*echfcs* using oligonucleotides
   p6apraMCS_for (5'-GCGAGCACCGGAGGCAGG-3') and
   p6apraMCS_rev (5'-CATTCATCCGGGGTCAGCAC-3')
(b9) cloning the blunt PCR product in the *Hinc*II-linearized suicide plasmid p6suigusA::Δ*vdh*, to achieve an integration at the Δ*vdh-*locus;
(b10) transferring the resulting plasmid p6suiΔ*vdh*::permE*::*echfcs* to *Amycolatopsis* sp. ATCC 39116 Δ*vdh*.
(b11) selecting transformants of *Amycolatopsis* sp. ATCC 39116 with regard to correct genotype and cultivating them to obtain a second recombinant event,
   which convert ferulic acid into vanillin.

Another object of the present invention relates to a process for preparing vanillin comprising subjecting ferulic acid to Amycolatopsis sp. ATCC 39116 - mutant *Amycolatopsis* sp. ATCC 39116 Δ*vdh* which is F33 as defined in Claim 1 or mutant *Amycolatopsis* sp. ATCC 39116 Δ*vdh::permE*::echfcs* which is F84 as defined in Claim 1 which converts ferulic acid into vanillin, for a period of time sufficient to convert said ferulic acid to vanillin, and recovering the vanillin thus formed.

Preferably said ferulic acid is natural ferulic acid.

Also preferred is a process, wherein said microorganism is used to convert ferulic acid to vanillin and the microorganism is contained in a medium which comprises a carbon source. Particular preferred are carbon sources selected from the group consisting of sugars, sugar alcohols, organic acids and complex mixtures. It is also helpful using a medium that further comprises a nitrogen source.

Another object of the present invention refers to a process comprising using vanillin as a flavouring, wherein the improvement comprises using as said vanillin the vanillin produced by a process as described above, preferably involving vanillin that is produced from natural ferulic acid.

According to processes known from the prior art using conventional *Amycolatopsis* sp. ATCC 39116 strains, a final concentration of 13.9 g/l and a molar yield of 75 % vanillin could be obtained. Surprisingly, it has been observed that using the improved strains an end concentration of 17.5 g/l (F33) and 19.3 g/l (F84) was achieved, which means an improvement of up to 72 %.

### BRIEF DISCUSSION OF THE RESULTS OBTAINED USING STRAIN F33

To engineer the metabolism of *Amycolatopsis* sp. ATCC 39116 strains towards a higher productivity in fermentations processes, markerless gene deletion mutant of *vdh* was generated, coding for the above mentioned vanillin dehydrogenase, in order to interrupt the catabolism of the target product. For this, the suicide plasmid p6suigusA was established, that does not replicate in *Amycolatopsis* sp. ATCC 39116 and must therefore integrate into the genome to be further propagated. Due to illegitimate recombination events that often occur in actinomycetes, only 1% of the analysed mutants showed a correct homologous recombination at the desired locus in the genome. As integration events of the basic vector p6sui, lacking the *gusA* gene, were very seldom, it is assumed that recombination events between this gene and the genome of *Amycolatopsis* sp. ATCC 39116 took place.

Nevertheless, the system was found particular useful for the phenotypic identification of hetero- and homogenotes, which considerably simplified the screening of potential mutants as known from the state of the art for other actinomycetes.

Finally, the system represents a powerful tool to generate a knockout of the targeted gene without introduction of heterologous DNA. Such a gene deletion system is unknown so far and can be applied for the introduction of knockouts in *Amycolatopsis* sp. ATCC 39116.

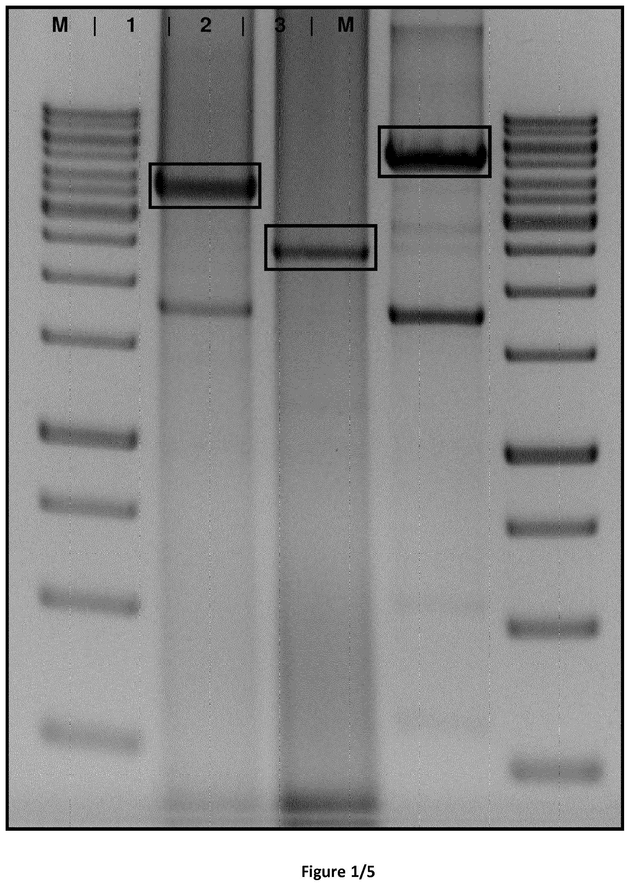

The gene deletion could be verified by diagnostic PCR as well as by sequencing of the genomic locus (**Figure 1**). Based on our knowledge of previous investigations, we examined the behavior of the new mutant strain *Amycolatopsis* sp. ATCC 39116 Δ*vdh* (Strain F33) regarding the accumulation and subsequent catabolism of vanillin in comparison to the wild type which is until now used in industry.

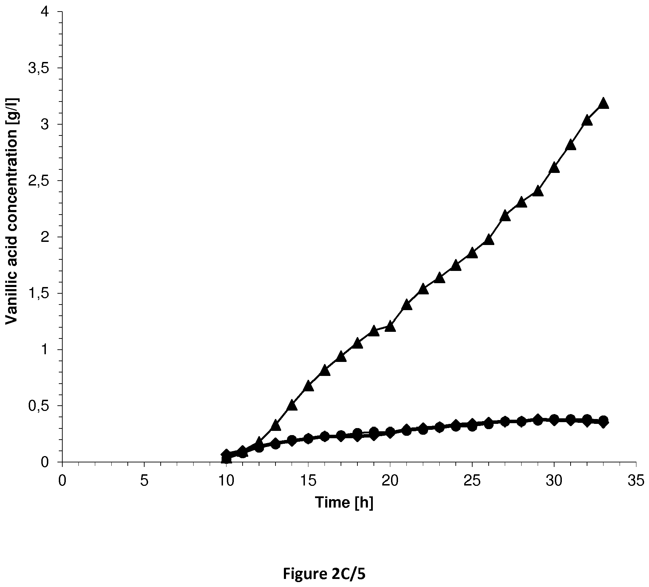

The fermentation experiments confirmed the observations that we already made during the previous biotransformation experiments. The catabolism of vanillin in mutant F33 was decreased by nearly 90%. While *Amycolatopsis* sp. ATCC 39116 accumulated vanillic acid up to a concentration of 3.2 g/l, due to the continuous degradation of vanillin, the *vdh-*deletion mutant only showed a residual catabolism, which led to a vanillic acid concentration of 0.34 g/l (**Figure 2C**). Further metabolic engineering might even improve this effect since aldehyde dehydrogenase or oxidase activities seem still to be present even for *Amycolatopsis* sp. ATCC 39116. As a matter of fact, the knockout of *vdh* is considered to be the crucial step of a consistent metabolic engineering to stop or at least to decrease the degradation of the desired product.

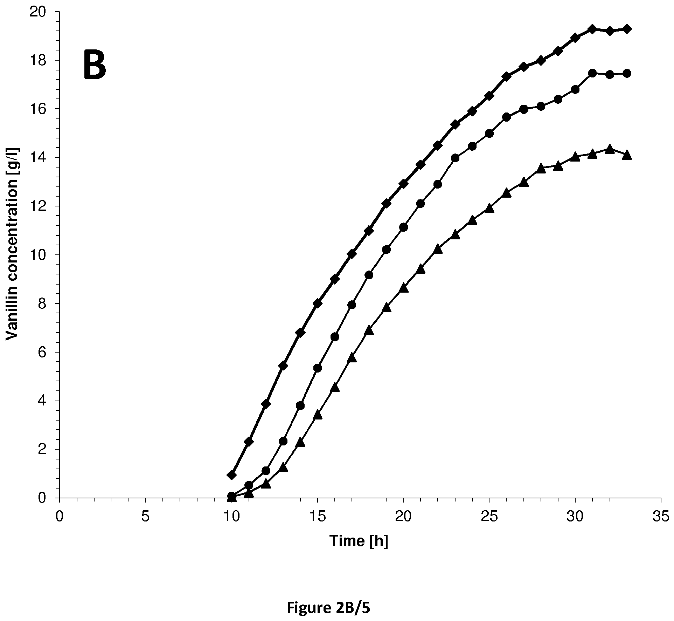

In accordance to these observations vanillin was accumulated directly and with an enhanced rate by F33 (**Figure 2B**). While vanillic acid is the first overflow product of the ferulic acid catabolism of *Amycolatopsis* sp. ATCC 39116, the *vdh* deletion mutant F33 accumulates vanillin directly without an initial synthesis of vanillic acid. This relationship between initial vanillic acid synthesis and subsequent vanillin accumulation is known for the wild type from other studies, in which the strain was used in fermentation experiments or where vanillic acid was shown to enhance the synthesis of vanillin in flask experiments.

It was observed that reduced catabolism resulted in a significantly higher end concentration with an even higher molar yield of vanillin. F33 accumulated vanillin at a maximal rate of 1.4 g/l/h (wild type: max. 1.15 g/l/h). The enhanced synthesis led to an end concentration of 17.5 g/l (**Table 2**), which is an improvement of more than 3 g/l compared to the wild type cells (Table 2: 14.1 g/l). Moreover, strain F33 metabolized ferulic acid to vanillin with a molar yield of 91.0%, which is an improvement of more than 16 percentage points with respect to the state of the art.

### BRIEF DISCUSSION OF THE RESULTS OBATINED USING STRAIN F84

To avoid catabolism of the substrate and to optimize the metabolic network of *Amycolatopsis* sp. ATCC 39116 Δ*vdh* the new strain F33 was further improved *by integration of* additional copies of the genes *ech* and *fcs* into the genome under the control of the constitutive promoter *permE*.* The genes encode the vanillin anabolism related enzymes feruloyl-CoA synthetase and enoyl-CoA hydratase/aldolase.

The overexpression construct was integrated at the former *vdh* locus, employing the same strategy with the use of p6suigusA as mentioned before. Although some illegitimate integration events of the suicide plasmid took place, finally a mutant was generated, with an integration of fragment *permE**::*ech*::*fcs* in the genome. The genomic organization was again verified by diagnostic PCR (Figure 1) and sequencing of the yielded amplificate.

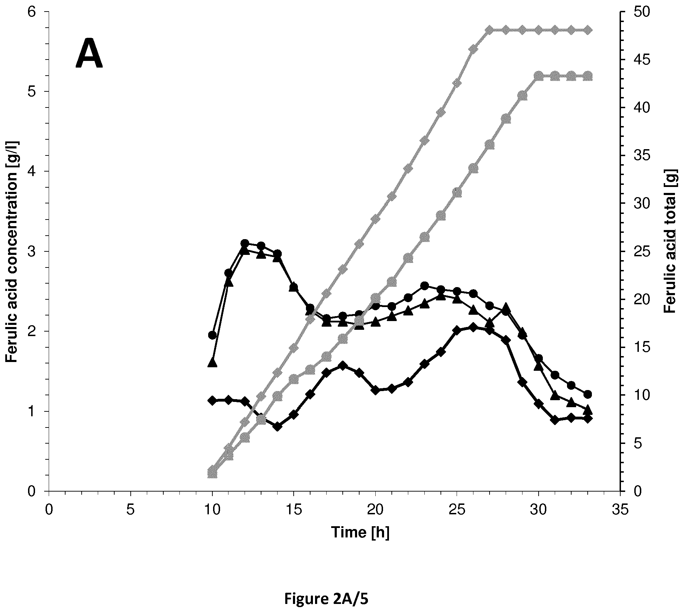

The new mutant *Amycolatopsis* sp. ATCC 39116 Δ*vdh::permE*::echfcs* (strain F84) was consequently also tested under the same fermentation conditions like F33 and the wild type. In comparison to the other two strains, the catabolism of ferulic acid by F84 was strongly enhanced, which was obvious due to considerably lower concentrations of the substrate in the medium, although ferulic was fed with a higher rate throughout the whole fermentation (**Figure 2A**). Thereby a higher amount of substrate can be fed within a shorter period, before the catabolism of ferulic acid ceases at the end of the fermentation.

Furthermore, the constitutive expression of *ech* and *fcs* resulted in a substantially enhanced synthesis of vanillin, especially during the first 8 hours of the biotransformation phase. The previously observed adaption phase of the wild type could be completely overcome as 1.6 g/l/h of vanillin were synthesized directly after supplementation of ferulic acid (Figure 2B).

By this also the total yield of the desired product could be increased to 19.3 g/l with a total molar vanillin yield of 92.0 %, with respect to added substrate. Given that 0.91 g/l of ferulic acid remained in the medium at the end of the fermentation, the strain metabolized the used substrate with a molar yield of 96.2%, which nearly approaches the theoretical possible value.

As a matter of fact F84 showed an even slightly reduced accumulation of vanillic acid in comparison to F33 although both strains do not express Vdh_{ATCC 39116} due to the deletion of the corresponding gene (**Figure 2C**). As the formation of vanillic acid by the deletion mutants seems not to be related to the amount of vanillin, but somehow to the catabolism of ferulic acid, it is assumed that vanillic acid is synthesized through a vanillin-independent pathway. In this coenzyme-A-dependent, β-oxidative deacetylation pathway, ferulic acid is first activated by Fcs, and then further processed by a homologue of Ech. In contrast to the non β-oxidative pathway, that yields vanillin in *Amycolatopsis* sp. ATCC 39116, the common intermediate 4-hydroxy-3-methoxyphenyl-β-hydroxypropionyl-CoA, is further converted via vanillyl-CoA to vanillic acid, without formation of vanillin.

The enhanced expression of *ech* in F84 is considered to result in a directed formation of vanillin, while less 4-hydroxy-3-methoxyphenyl-β-hydroxypropionyl-CoA can be converted to vanillic acid. This assumption is also supported by the observation that the formation of vanillic acid is retarded, when the catabolism of ferulic acid decreases at the end of the fermentation. On the other hand vanillic acid is continuously formed by the wild type from vanillin due to the inherent activity of the vanillin dehydrogenase.

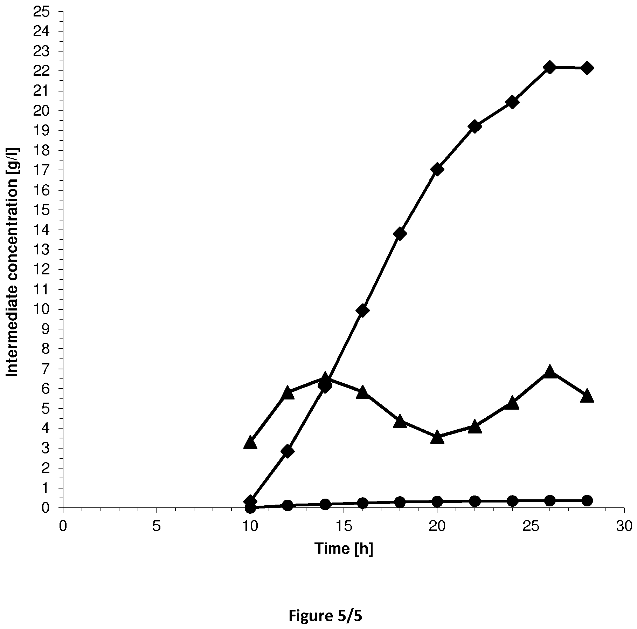

The presented fermentation data constitute the best overall productivity for all shown strains, with regard to the final vanillin concentration, the molar product yield and the run time of the whole fermentation. By increasing the feeding rate, a shorter runtime can be achieved, yielding comparable final concentrations, but lower molar product yields, considering the amount of ferulic acid, that was fed. On the other hand, higher molar yields of up to 96.4% can be reached using strain F84, if the desired final vanillin concentration and therefore the amount of supplied ferulic acid were lower. Furthermore, we were able to produce 22.3 g/l of vanillin, if ferulic acid was supplied at a rate of more than 5.5 g/h (**Figure 5**).

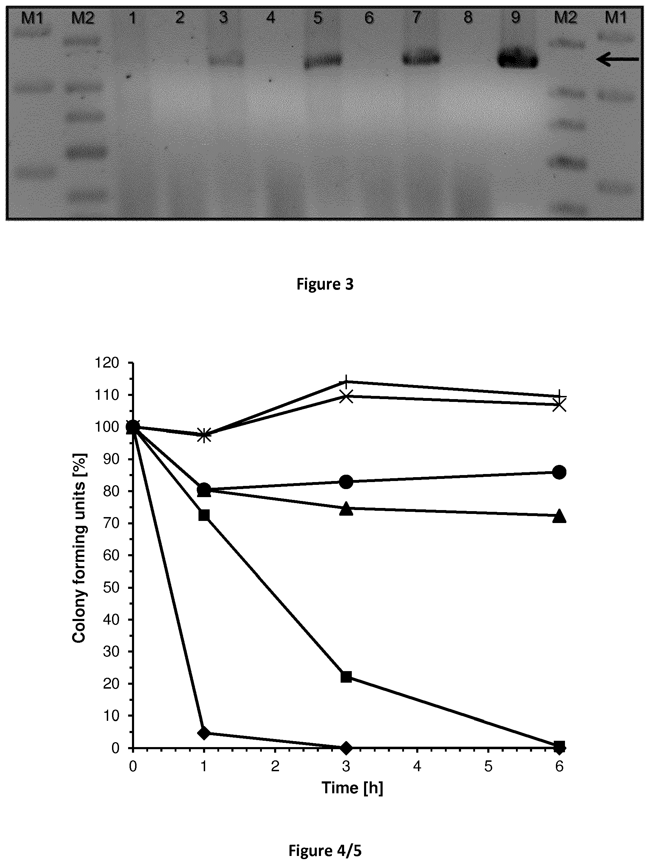

In conclusion, the synthesis of vanillin by the fermentation process according to the present invention seems to be limited to the antimicrobial properties of the product, although higher final concentrations can be achieved, at the expense of a lower total yield, by variation of some process parameters. The vanillin toxicity tests clearly showed the antibacterial effect of vanillin on the cells (**Figure 4**). Although the cells seem to tolerate higher vanillin concentrations than 10 g/l in the fermentation processes, the catabolism of ferulic acid stops at the end of the cultivation and the fermentation parameters clearly indicated that the cell metabolism collapses. Since the activation of ferulic acid is ATP- and CoA dependent, a further degradation might be limited to the availability of cofactors. This is also supported by the fact, that supplementation of glucose with the feeding solution, in order to provide an additional source of carbon and energy, led to higher end concentrations and an enhanced rate of the catabolism.

However, phenolic compounds, like vanillin, have been shown to effect or even inhibit DNA, RNA and protein synthesis, the uptake of glucose, pH homeostasis and ion gradients as they most likely target the cytoplasmic membrane due to their hydrophobic nature. Moreover, the aldehyde group was shown to even enhance this toxic effect, since it shows high reactivity and can form covalent bonds with DNA and proteins. A more increased vanillin formation is therefore thought to be limited to the viability of the cells. Although the enzymes might still be active at the end of the fermentation, further catabolism should be hampered due to the unavailability of cofactors. The extraction of vanillin in order to keep a non-toxic concentration was shown to enhance the overall yield of a production process and is suggested to overcome this problem.

### PROCESSES FOR OBTAINING THE MUTANTS F33 AND F84

Two further embodiments of the present invention relate to processes for obtaining the *Amycolatopisis* mutants F33 and F84

### STEP 1

The first step which is common for both processes according to the present invention refers to the generation of a suicide plasmid for the generation of a markerless deletion mutant of *Amycolatopsis* sp. ATCC 39116, encompassing the following steps:
- The *E. coli* - *Amycolatopsis* shuttle vector p6apra was digested with *Nae*I/*Sac*II to eliminate the essential part of the origin of replication for *Amycolatopsis.*
- The resulting fragment (3575 bp) contained *oriV* for replication in *E. coli,* the apramycin resistance gene and several unique restriction sites for cloning.
- The fragment was religated and *E. coli* was transformed with the resulting plasmid p6sui.
- Subsequently, the glucuronidase gene *gusA* was isolated as SpeI/*Eco*RV fragment from pSETGUS and cloned into the *Nhe*I/*Dra*I-linearized suicide plasmid p6sui resulting in p6suigusA.

### STEP 2

The second step refers to the mutant construction F33 (including deletion of vanillin dehydrogenase gene *vdh*) encompassing the following steps:
- A precise deletion of the *vdh* gene was accomplished via homologous recombination. The flanking regions upstream and downstream of *vdh* in the genome of *Amycolatopsis* sp. ATCC 39116 were amplified using the following oligonucleotides:
   upstream region:
      *vdh*LF*_*for3 (5'-TCGTACTTCGCGGTGATCTCGTGG-3')
         and
      *vdh*LF_rev_RF (5'-GCGCATCAGGAGTGACACGCGCGGCGGGGGCG-3');
   downstream region:
      *vdh*RF_for_LF (5'-CGCCCCCGCCGCGCGTGTCACTCCTGATGCGC-3') and
      *vdh*RF_rev3 (5'-TTGCAGTCCTTTGTAGAGCGACACG-3').
- The resulting amplificates were purified and combined in a subsequent fusion-PCR using primers *vdh*LF*_*for3 and *vdh*RF_rev3.
- The resulting fragment Δ*vdh* combined the upstream and downstream regions of *vdh* and was cloned into the *Eco*RV-site of the suicide plasmid p6suigusA.
- After isolation of a resulting plasmid p6suigusA::Δ*vdh* from *E. coli,* the vector was transferred to *Amycolatopsis* sp. ATCC 39116.
- Transformants of *Amycolatopsis* sp. ATCC 39116 were selected on solid Caso medium containing 50 µg/ml apramycin. Additionally, the resulting colonies were overlaid with X-Gluc as previously described to detect β-glucuronidase activity encoded by the suicide plasmid (Myronovskyi et al. 2011).
- Mutants that showed the desired phenotype were first screened for the correct genomic integration of the suicide plasmid p6suigusA::Δ*vdh* by colony-PCR using primers *vdh*LF*_*for3 and *vdh*RF_rev3, which bind upstream and downstream of the recombination regions, respectively.
- Afterwards, mutants that showed the correct genotype were cultivated in liquid Caso medium to obtain a second recombination event.
- For this the cells were cultivated without antibiotics for at least 10 passages and were subsequently diluted and plated on Caso agar plates. Single colonies that did not show glucuronidase activity after overlaying with X-Gluc were picked on agar plates with and without apramycin.
- Mutants, which had lost the apramycin resistance, were finally analyzed via diagnostic PCR with different primer combinations showing a correct deletion of *vdh.* Furthermore, the resulting PCR-fragments were analyzed via digestion and sequencing to verify the genotype of *Amycolatopsis* sp. ATCC 39116 Δ*vdh.*

Therefore, the process for obtaining mutant F33 can be summarized as follows:
(a1) digesting *E. coli* - *Amycolatopsis* shuttle vector p6apra with *Nae*I/*Sac*II to eliminate the essential part of the origin of replication for *Amycolatopsis* and to obtain the resulting fragment (3575 bp) containing *oriV* for replication in *E. coli,* the apramycin resistance gene and several unique restriction sites for cloning;
(a2) religating the fragment and transforming *E. coli* with the resulting plasmid p6sui;
(a3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(a4) deleting of the *vdh* gene by homologous recombination, wherein the flanking regions upstream and downstream of *vdh* in the genome of *Amycolatopsis* sp. ATCC 39116 are amplified using the following oligonucleotides:
   upstream region:
      *vdh*LF_for3(5'-TCGTACTTCGCGGTGATCTCGTGG-3')
         and
      *vdh*LF_rev_RF (5'-GCGCATCAGGAGTGACACGCGCGGCGGGGGCG-3');
   downstream region:
      *vdh*RF_for_LF (5'-CGCCCCCGCCGCGCGTGTCACTCCTGATGCGC-3') and
      vdhRF_rev3 (5'-TTGCAGTCCTTTGTAGAGCGACACG-3');
(a5) purifiying the resulting amplificates and combining them in a subsequent fusion-PCR using primers *vdh*LF*_*for3 and *vdh*RF_rev3;
(a6) cloning the resulting fragment Δ*vdh* including the upstream and downstream regions of *vdh* into the *Eco*RV-site of the suicide plasmid p6suigusA;
(a7) isolating the resulting plasmid p6suigusA::Δ*vdh* from *E. coli* and transferring the vector to *Amycolatopsis* sp. ATCC 39116; and
(a8) selecting transformants of *Amycolatopsis* sp. ATCC 39116 with regard to correct genotype and cultivating them to obtain a second recombinant event;

### STEP C

- Step C covers the mutant construction F84 (integration of additional gene copies of *ech* and *fcs* at former *vdh* locus) and encompasses the following steps:
- Based on *Amycolatopsis* sp. ATCC 39116 Δ*vdh* a further mutant strain was constructed, which carries additional copies of the genes *ech* and *fcs* under the control of the strong constitutive promoter *permE*;*
- The genes *ech* and *fcs* were amplified together from genomic DNA of *Amycolatopsis* sp. ATCC 39116 using oligonucleotides
   ∘ *ech_*for*_Sac*I (5'-AAAAGAGCTCTAAGGAGGTGACAACTGCTGGC CGCGCTCG-3') and
   ∘ *fcs_rev_Xba*I (5'-AAAATCTAGACCACAGAGTAGCCGCAGCGGG-3').
- The PCR product was digested with *Sac*I and *Xba*I and subsequently cloned into the *Sac*I/*Xba*I-linearized pBluescript SK⁻. The resulting plasmid *pSK::echfcs* was transferred to *E. coli* Mach-1 T1 and enzyme activity was observed in ferulic acid biotransformation experiments.
- Next, *pSK::echfcs* was digested with *Eco*ICRI and *Hind*III*,* and the resulting fragment, which contained both genes, was ligated with the *Fsp*AI/*Hin*dIII-linearized expression vector p6permE, collinear to *permE** (Fleige and Steinbüchel 2014).
- For the integration into the genome of *Amycolatopsis* sp. ATCC 39116 Δ*vdh* a fragment, comprising the promoter *permE** and the genes *ech and fcs,* was amplified from the resulting plasmid p6permE::*echfcs* using oligonucleotides
   ∘ p6apraMCS_for (5'-GCGAGCACCGGAGGCAGG-3') and
   ∘ p6apraMCS_rev (5'-CATTCATCCGGGGTCAGCAC-3').
- The blunt PCR product was cloned in the *Hinc*II-linearized suicide plasmid p6suigusA::Δ*vdh*, to achieve an integration at the Δ*vdh*-locus.
- The resulting plasmid was designated p6suiΔ*vdh*::*permE**::*echfcs* and transferred to *Amycolatopsis* sp. ATCC 39116 Δ*vdh.*
- Screening of transformants, generation of the second recombination event and verification of the correct integration of the overexpression construct at the Δ*vdh-*locus was performed as described above.

Therefore, the process for obtaining mutant F84 can be summarized as follows:
(b1) digesting *E. coli* - *Amycolatopsis* shuttle vector p6apra with *Nae*I/*Sac*II to eliminate the essential part of the origin of replication for *Amycolatopsis* and to obtain the resulting fragment (3575 bp) containing *oriV* for replication in *E. coli,* the apramycin resistance gene and several unique restriction sites for cloning;
(b2) religating the fragment and transforming *E. coli* with the resulting plasmid p6sui;
(b3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(b4) amplifying the genes *ech* and *fcs* together from genomic DNA of *Amycolatopsis* sp. ATCC 39116 using oligonucleotides
   *ech_*for*_Sac*I (5'-AAAAGAGCTCTAAGGAGGTGACAACTGCTGGC CGCGCTCG-3')
      and
   *fcs*_rev_*Xba*I (5'-AAAATCTAGACCACAGAGTAGCCGCAGCGGG-3');
(b5) digesting the PCR product with *Sac*I and *Xba*I and subsequently cloning it into the *Sac*I/*Xba*I-linearized pBluescript SK⁻.
(b6) transferring the resulting plasmid *pSK::echfcs* to *E. coli* Mach-1 T;
(b7) digesting *pSK::echfcs* with *Eco*ICRI and *Hind*III*,* and ligating the resulting fragment, containing both genes, with the *Fsp*AI/*Hin*dIII-linearized expression vector p6permE, collinear to *permE*;*
(b8) amplifying a fragment, comprising the promoter *permE** and the genes *ech* and *fcs* from the resulting plasmid p6permE::*echfcs* using oligonucleotides
   p6apraMCS_for (5'-GCGAGCACCGGAGGCAGG-3') and
   p6apraMCS_rev (5'-CATTCATCCGGGGTCAGCAC-3')
(b9) cloning the blunt PCR product in the *Hinc*II-linearized suicide plasmid p6suigusA::Δ*vdh*, to achieve an integration at the Δ*vdh-*locus;
(b10) transferring the resulting plasmid p6suiΔ*vdh*::*permE**::*echfcs* to *Amycolatopsis* sp. ATCC 39116 Δ*vdh.*
(b11) selecting transformants of *Amycolatopsis* sp. ATCC 39116 with regard to correct genotype and cultivating them to obtain a second recombinant event.

### BRIEF DESCRIPTION OF THE FIGURES

### FIGURE 1

### Verification of the gene replacement in Amycolatopsis sp. ATCC 39116 Δvdh (F33) and Amycolatopsis sp. ATCC39116Δvdh::permE*::echfcs (F84)

In order to verify the correct gene deletion of *vdh* and the subsequent integration of *permE*::echfcs,* a diagnostic PCR was carried out using chromosomal DNA as template. The resulting fragments were analyzed in an 1 % agarose gel. Molecular weight marker (**M**): "GeneRuler™ 1 kb DNA Ladder" (Fermentas GmbH, St. Leon-Rot, Germany). Lanes 1, 2 and 3 represent the amplificate using oligonucleotides *vdh*LF_for_diag and *vdh*RF_rev2, which show the difference between the wild type (**1**, 3.7 kb), mutant F33 (**2**, 2.4 kb) and mutant F84 (**3**, 5.4 kb). The marked fragments were purified, and the amplificates were analyzed by sequencing to finally verify the correct homologous recombination.

### FIGURE 2A, 2B, 2C

### Biotransformation of ferulic acid by Amycolatopsis sp. ATCC 39116 and the related mutants F33 and F84

Cells were grown in 1000 ml fermentation broth in a 2I-bioreactor at 45 °C until they reached the stationary growth phase. Subsequently, the feeding with ferulic acid solution was started. Biotransformation of ferulic acid (**A**) to vanillin (**B**) and vanillic acid (**C**) was observed at the given times using HPLC analyses as described in materials and methods. Grey curves in diagram **A** represent the total amount of ferulic acid that was fed. Black curves represent the concentrations of the intermediates in the fermentation broth. (▲,▲) *Amycolatopsis* sp. ATCC 39116; (●,●) mutant F33; (◆,◆) mutant F84

### FIGURE 3

### RT-PCR analysis of ech expression

The expression of *ech and fcs* in different sections of the fermentation was analyzed by RT-PCR. Samples were taken prior to feeding with ferulic acid and after 7 hours of biotransformation. RNA isolation, preparation of cDNA and the final PCR were performed as described in materials and methods. The resulting fragments were analyzed in a 2 % agarose gel. Molecular weight marker **M1:** "GeneRuler™ 1 kb DNA Ladder"; **M2** : "GeneRuler™ Low Range DNA Ladder" (Fermentas GmbH, St. Leon-Rot, Germany)
Lane 1 to 9 represent the amplificates using oligonucleotides *ech*_RT_for and *fcs* RT_rev and different templates: **1:** cDNA, prepared from F33 sample prior to feeding; **2:** RNA control for sample 1; **3:** cDNA, prepared from F33 sample during feeding; **4:** RNA control for sample 3; **5:** cDNA, prepared from (F84) sample prior to feeding; **6:** RNA control for sample 5; **7:** cDNA, prepared from F84 sample during feeding; **8:** RNA control for sample 7; **9:** DNA-control for PCR. The arrow indicates the expected internal 624 bp-fragment of the transcript of *ech* and *fcs.*

### FIGURE 4

### Vanillin toxicity test

Cells of *Amycolatopsis* sp. ATCC 39116 were cultivated in 300 ml Caso medium at 45°C until the culture reached the stationary growth phase. Samples were taken, diluted with sterile saline (0.9 % NaCl) and plated on solid Caso medium in triplicates to determine the starting cell density (100%). Six equal aliquots of 45 ml were prepared. Vanillin (dissolved in DMSO) was added in concentrations of 2 (●), 4 (▲), 7 (■) and 10 g/l (◆). As control experiments an equal amount of DMSO was added to one of the cultures (+), while another culture was monitored without any addition (**x**). The cultures were further incubated at 45°C and samples were again taken, diluted and plated on agar plates after 1, 3 and 6 hours. Colony forming units were counted again and the percentages were calculated with regard to the starting cell density.

### FIGURE 5

### Biotransformation of ferulic acid by F84

Cells were grown in 1000 ml fermentation broth in a 2I-bioreactor at 45 °C until they reached the stationary growth phase. Subsequently, the feeding with ferulic acid solution (5.5 g/l/h ferulic acid) was started. Biotransformation of ferulic acid (▲) to vanillin (◆) and vanillic acid (●) was observed at the given times using HPLC analyses as described in materials and methods. The curves represent the concentrations of the intermediates in the fermentation broth.

### EXAMPLES

### MATERIALS AND METHODS

### BACTERIAL STRAINS, PLASMIDS AND CULTIVATION CONDITIONS

All bacterial strains and plasmids used in this study are listed in following **Table 1.**

**Table 1**

| Bacterial strains and plasmids used in this study | | |
|---|---|---|
| **Bacterial strains and plasmids** | **Description, sequence** | **Reference** |
| **Strains** | | |
| *Amycolatopsis* sp. ATCC 39116 | wild type | American Type Culture Collection, No. 39116 |
| *Amycolatopsis* sp. ATCC 39116 Δ*vdh* (F33) | *vdh* deletion mutant of *Amycolatopsis* sp. ATCC 39116 | Present application |
| *Amycolatopsis* sp. ATCC 39116 Δ*vdh*:: *permE**::*echfcs* (F84) | Δ*vdh,* additional copies of *ech* and *fcs* under control of *permE** promoter | Present application |
| *E. coli* Mach-1 T1 | F ⁻ φ80(*lac*Z)ΔM15 Δ*lacX*74 *hsdR* (rₖ⁻ mₖ⁺) Δ*recA*1398 *endA1 tonA* | Invitrogen GmbH |
| *E. coli* K12 ER 2925 | *ara-14 leuB6 fhuA31 aclY1 tsx78 glnV44 galK2 galT22 mcrA dcm-6 hisG4 rfbD1 R*(*zgb210*::*Tn10*)*TetS endA1 rpsL136 dam13*::*Tn9 xylA-5 mtl-1 thi-1 mcrB1 hsdR2* | New England Biolabs Inc. (Ipswich, MA, USA) |

| **Plasmids** | | |
|---|---|---|
| p6apra | Apra^{r} from pMA5096, *pArep, oriV* | (1) |
| pBluescript SK-(pSK-) | Ap^{r}, *LacPOZ',* T7 and T3-promoter | Agilent GmbH, Waldbronn (DE) |
| p6permE | p6apra with *ermE**-promoter | (1) |
| pSETGUS | *Streptomyces* integrative vector, *gusA* under control of *tipA*-promoter | (2) |
| p6sui | *Nae*I/SacII-religated p6apra lacking the essential part of *pArep,* suicide-plasmid | Present application |
| p6suigusA | *Spe*I/*Eco*RV fragment of pSETGUS containing *gusA* cloned into *Nhe*I/*Dra*I-sites of p6sui | Present application |
| p6suigusA::Δ*vdh* | p6suigusA with flanking region of *vdh,* suicide plasmid for knockout of *vdh* | Present application |
| p6suigusA::Δ*vdh:: permE*::ech::fcs* | p6suigusA::Δ*vdh* with overexpression construct for *ech* and *fcs* under control of *permE**promoter | Present application |

| | | |
|---|---|---|
| (1) Appl. Environ. Microbiol 77: 5370-5283 (2014) (2) *ibid.* 77:5370-5283 (2011) | | |

Cells of *Escherichia coli* were grown in Lysogeny Broth (LB) medium at 37 °C. Cells of *Amycolatopsis* sp. ATCC 39116 were grown at 45 °C in Caso medium (Merck, Darmstadt, Germany). For selection of plasmid harboring strains, antibiotics were added to the medium as follows: Ampicillin: 100 µg/ml for *E. coli;* Apramycin: 50 µg/ml for *E. coli* and *Amycolatopsis.* Cell growth was measured by determination of the optical density at 400 nm (*Amycolatopsis* sp. ATCC 39116) or 600 nm (*E. coli*).

### DNA ISOLATION AND MODIFICATION

Plasmid DNA was isolated from *E. coli* using the "peqGOLD Plasmid MiniPrep Kit I" (PEQLAB GmbH, Erlangen, Germany). DNA was digested by restriction endonucleases (Thermo Fisher Scientific GmbH, Schwerte, Germany) under the conditions described by the manufacturer. Phusion Hot-Start II high-fidelity DNA polymerase, T4 DNA ligase, T4 DNA polymerase and RevertAid Reverse Transcriptase (Thermo Fisher Scientific GmbH, Schwerte, Germany) were used according to the instructions of the manufacturer. Oligonucleotides were purchased from Eurofins MWG Synthesis GmbH (Ebersberg, Germany). DNA fragments were isolated from agarose gels or reaction mixtures using the "peqGOLD Gel Extraction Kit" (PEQLAB GmbH, Erlangen, Germany).

### TRANSFER OF DNA

Plasmids were transferred into *E. coli* by employing the CaCl₂-method, whereas the transfer of plasmids into *Amycolatopsis* sp. ATCC 39116 was performed by direct mycelia transformation as previously described.

### PLASMID AND MUTANT CONSTRUCTION

For the generation of a markerless gene deletion mutant of *Amycolatopsis* sp. ATCC 39116 a suicide plasmid was constructed. The previously reported *E. coli* - *Amycolatopsis* shuttle vector p6apra was digested with *Nae*I/*Sac*II to eliminate the essential part of the origin of replication for *Amycolatopsis.* The resulting fragment (3575 bp) still contained *oriV* for replication in *E. coli,* the apramycin resistance gene and several unique restriction sites for cloning. The fragment was religated and *E. coli* was transformed with the resulting plasmid p6sui. Subsequently, the glucuronidase gene *gusA* was isolated as *spe*I/*Eco*RV fragment from pSETGUS (21) and cloned into the *Nhe*I/*Dra*I-linearized suicide plasmid p6sui resulting in p6suigusA.

A precise deletion of the *vdh* gene was accomplished via homologous recombination. The flanking regions upstream and downstream of *vdh* in the genome were amplified using the following oligonucleotides:
Upstream region:
   *vdh*LF*_*for3 (5'-TCGTACTTCGCGGTGATCTCGTGG-3') and
   *vdh*LF_rev_RF (5'-GCGCATCAGGAGTGACACGCGCGGCGGGGGCG-3');
Downstream region:
   *vdh*RF_for_LF (5'-CGCCCCCGCCGCGCGTGTCACTCCTGATGCGC-3') and
   *vdh*RF_rev3 (5'-TTGCAGTCCTTTGTAGAGCGACACG-3').

The resulting amplificates were purified and combined in a subsequent fusion-PCR using primers *vdh*LF_for3 and *vdh*RF_rev3. The resulting fragment Δ*vdh* combined the upstream and downstream regions of *vdh* and was cloned into the *Eco*RV-site of the suicide plasmid p6suigusA. After isolation of a resulting plasmid p6suigusA::Δ*vdh* from *E. coli,* the vector was transferred to *Amycolatopsis* sp. ATCC 39116. Transformants of *Amycolatopsis* sp. ATCC 39116 were selected on solid Caso medium containing 50 µg/ml apramycin. Additionally, the resulting colonies were overlaid with X-Gluc as previously described to detect β-glucuronidase activity encoded by the suicide plasmid [see: Appl. Environ. Microbiol. 77:5370-5283 (2011**)]**

Mutants that showed the desired phenotype were first screened for the correct genomic integration of the suicide plasmid by colony-PCR using primers *vdh*LF_for3 and *vdh*RF_rev3, which bind upstream and downstream of the recombination regions, respectively. Afterwards, mutants that showed the correct genotype were cultivated in liquid Caso medium to obtain a second recombination event. For this the cells were cultivated without antibiotics for at least 10 passages and were subsequently diluted and plated on Caso agar plates. Single colonies that did not show glucuronidase activity after overlaying with X-Gluc were picked on agar plates with and without apramycin. Mutants, which had lost the apramycin resistance, were finally analyzed via diagnostic PCR with different primer combinations showing a correct deletion of *vdh.* Furthermore, the resulting PCR-fragments were analyzed via digestion and sequencing to verify the genotype of *Amycolatopsis* sp. ATCC 39116 Δ*vdh.*

Based on *Amycolatopsis* sp. ATCC 39116 Δ*vdh* a further mutant strain was constructed, which carries additional copies of the genes *ech and fcs* under the control of the strong constitutive promoter *permE** (18). The genes *ech* and *fcs* were amplified together from genomic DNA of *Amycolatopsis* sp. ATCC 39116 using oligonucleotides:
(i) *ech_*for*_Sac*I (5'-AAAAGAGCTCTAAGGAGGTGACAACTGCTGGCCGCGCTCG-3') and
*(ii) fcs_rev_Xba*I (5'-AAAATCTAGACCACAGAGTAGCCGCAGCGGG-3').

The PCR product was digested with *Sac*I and *Xba*I and subsequently cloned into the *Sac*I/*Xba*I-linearized pBluescript SK⁻. The resulting plasmid *pSK::echfcs* was transferred to *E. coli* Mach-1 T1 and enzyme activity was observed in ferulic acid biotransformation experiments. Next, *pSK::echfcs* was digested with *Eco*ICRI and *Hin*dIII*,* and the resulting fragment, which contained both genes, was ligated with the *Fsp*AI/HindIII-linearized expression vector p6permE, collinear to *permE*.* For the integration into the genome of *Amycolatopsis* sp. ATCC 39116 Δ*vdh* a fragment, comprising *permE** and the genes *ech* and *fcs,* was amplified from the resulting plasmid p6permE::*echfcs* using oligonucleotides p6apraMCS_for (5'-GCGAGCACCGGAGGCAGG-3') and p6apraMCS_rev (5'-CATTCATCCGGGGTCAGCAC-3'). The blunt PCR product was cloned in the *Hinc*II-linearized suicide plasmid p6suigusA::Δ*vdh*, to achieve an integration at the Δ*vdh*-locus.

The resulting plasmid was designated p6suiΔ*vdh*::*permE**::*echfcs* and transferred to *Amycolatopsis* sp. ATCC 39116 Δ*vdh.* Screening of transformants, generation of the second recombination event and verification of the correct integration of the overexpression construct at the Δ*vdh*-locus was performed as described above

### GLUCURONIDASE ENZYME ASSAY

To detect β-glucuronidase activity on agar plates, the colonies were overlaid with 1ml of 1mM 5-bromo-4-chloro-3-indolyl-beta-D-glucuronic acid (X-Gluc) solution. After a short incubation period colonies containing hydrolytic GUS activity turned blue because of the formation of 5,5'-dibromo-4,4'-dichloro indigo.

### FED-BATCH FERMENTATION AT THE 2L-SCALE

Fed-batch fermentation experiments were carried out in a 2-liter Biostat B Plus (Sartorius, Göttingen, Germany) fermenter. Temperature, pH, dissolved oxygen (pO₂) and cell density (OD 850 nm) were monitored online during the fermentation. The cultivation was performed at 45° C with a fixed aeration rate of 1.0 vvm (volume per volume per minute) and a stirrer cascade between 300 and 600 rpm in order to keep a pO₂ saturation of 25%. The fermentation broth consisted of 4 g/l glucose and yeast extract, respectively. As antifoam agent 2 g/l of SAG 710 emulsion (Momentive Performance Materials Inc., Albany, USA) was added to the medium. The cells were grown until they reached the stationary growth phase, whereupon the biotransformation was started through continuous feeding of ferulic acid (stock solution: 800ml, 55 g/l, pH 8.0 NaOH, 4g/l glucose, 4g/l yeast extract). The feeding of ferulic acid was adjusted between a rate of 1.9 and 3.5 g/h to ensure an appropriate substrate supply during the biotransformation. The substrate concentration, as well as the concentration of intermediates of the catabolism were analyzed by HPLC at the given time points. The fermentation was stopped after 20-24h of biotransformation when further synthesis of vanillin had ceased.

### DETERMINATION OF METABOLIC INTERMEDIATES

Excreted intermediates of the ferulic acid metabolism were analyzed by high performance liquid chromatography using a "UltiMate 3000 HPLC System" (Dionex, Idstein, Germany) without prior extraction. Culture supernatants were obtained after centrifugation (5 min, 16,000 x g) and diluted 1:10 to 1:100 with double distilled water, if necessary. Intermediates were separated by reverse-phase chromatography using an Acclaim® 120 C8 column (particle size: 5 µm; column: 250 x 2.1 mm) with a gradient of 0.1% (vol/vol) formic acid (eluant A) and acetonitrile (eluant B) in a range from 15 to 100% (vol/vol) eluant B. The run started with a flow rate of 0.1 ml/min, which was raised to 0.3 ml/min after 16 min when eluent B reached 100 %. For quantification, all intermediates were calibrated with external standards. The compounds were identified by their retention times and their absorption at a specific wavelength determined using a multiple wavelength detector "MWD - 3000" (259 nm, 280 nm, 285 nm, 340 nm; Dionex, Idstein, Germany). Data analyses and quantifications were performed with the associated software "Chromeleon 6.8 Chromatography Data Systems" (Dionex, Idstein, Germany).

### VANILLIN TOXICITY TEST

Cells of *Amycolatopsis* sp. ATCC 39116 were cultivated in 300 ml Caso medium at 45°C until the culture reached the stationary growth phase. Samples were withdrawn, diluted with sterile saline (0.9 % NaCl) and plated on solid Caso medium in triplicates in order to count colony forming units for the determination of the starting cell density (100%). Subsequently, the culture was separated into six equal aliquots of 45 ml. Vanillin (dissolved in DMSO) was added in concentrations of 2, 4, 7 and 10 g/l. As control experiments an equal amount of DMSO was added to one of the cultures, while another culture was monitored without any addition. The cultures were further incubated at 45°C and samples were again taken, diluted and plated on agar plates after 1, 3 and 6 hours. Colony forming units were counted again and the percentages were calculated with regard to the starting cell density.

### RNA ISOLATION AND RT-PCR ANALYSIS

For the extraction of total RNA 1 ml samples of the fermentation broth were withdrawn and cells of the wild type *Amycolatopsis* sp. ATCC 39116 or mutant strains were harvested by centrifugation (13 000 x g, 20 min, 4°C). The resulting cell pellets were resuspended in 800 µl TE buffer (pH 8.0), and RNA was isolated as previously described. Remaining DNA contaminations were removed by the use of DNAse I (Roche, Mannheim, Germany), and RNA was subsequently purified as described before. Preparation of cDNA was accomplished using the RevertAid Reverse Transcriptase (Thermo Fisher Scientific GmbH, Schwerte, Germany) and oligonucleotides *ech*_RT_for (5'-GAACCCCACCCTGAACG-3') and *ech*_RT_rev (5'-GAGCTTCAGCGCCACCTC-3') for an internal fragment of *ech.*

For an overlapping fragment of *ech* and *fcs* oligonucleotides *echfcs_*RT*_*for (5'-GTACTACATCATGACCG GTGAGC-3') and ech*fcs*_RT_rev (5'-CAGCGCGTGGTCCAGTTC-3') were used. The final PCR was performed using Phusion Hot-Start II high-fidelity DNA polymerase. DNA contaminations in the isolated RNA were excluded in a control experiment with addition of RNA without the prior reverse transcription step.

### RESULTS

### CONSTRUCTION AND CHARACTERIZATION OF A VDH-DELETION MUTANT OF AMYCOLATOPSIS SP. ATCC 39116

The vanillin dehydrogenase VDH_{ATCC 39116} has a substantial impact on the catabolism of vanillin in *Amycolatopsis* sp. ATCC 39116 and is therefore a promising target to engineer the strains metabolism towards an efficient accumulation. After replacing the encoding gene *vdh* with a kanamycin resistance cassette it was intended to generate a markerless deletion mutant as new production strain. For this, a suicide plasmid was constructed that allowed the introduction of a suitable deletion construct and the screening of resulting heterogenotes. The origin of replication for *Amycolatopsis* of the shuttle vector p6apra was removed by digestion, yielding the non-replicating plasmid p6sui. The suicide plasmid still contained the origin of replication for *E. coli,* the apramycin resistance gene and several unique restriction sites for cloning of deletion constructs. To obtain the ability of phenotypic screening of homogenotes, the *gusA* gene of pSETGUS was inserted into p6sui, yielding p6suigusA. *GusA* encodes a β-glucuronidase, whose activity can easily be identified on agar plates, due to the strong blue colour of the colonies after overlaying them with X-Gluc. Homogenotes, which have lost the vector backbone, exhibited no colour.

For the deletion of *vdh* the flanking regions located upstream and downstream in the genome of *Amycolatopsis* sp. ATCC 39116 were amplified and subsequently combined in a fusion PCR. The resulting fragment Δ*vdh* was cloned in p6suigusA and the suicide plasmid p6suigusA::Δ*vdh* was transferred to *Amycolatopsis* sp. ATCC 39116. After the transfer, apramycin-resistant clones that exhibited glucuronidase activity could be observed. Unfortunately, we also received transformants after transfer of p6suigusA without the deletion construct in a control experiment. However, significantly more recombination events were observed when p6suigusA::Δ*vdh* was transformed. Because of these illegitimate recombination events the integration of p6suigusA::Δ*vdh* in the correct genomic locus was analyzed by diagnostic PCR at first. For this, the *vdh* locus was amplified and clones that included the suicide plasmid were identified through an 11.5 kb fragment. Afterwards two of the correct heterogenotes were cultivated in liquid Caso medium without added apramycin and samples where plated on Caso agar plates after at least 10 passages. Colonies which did not turn blue after X-Gluc overlaying were picked on solid Caso medium with and without apramycin. Clones, which had lost the apramycin resistance, were further analysed in a PCR reaction to identify a deletion of *vdh.* For this a fragment of the *vdh* locus was amplified using oligonucleotides, which bind further upstream and downstream of the used flanking regions, respectively. The correct deletion was demonstrated through amplification of a 2.42-kb fragment, which distinguishes in size from the wild type as a consequence of the loss of *vdh* (1,461 bp, **Figure 1**). Furthermore, the loss of *vdh* was confirmed by PCR employing internal primers, in order to demonstrate that the gene was not integrated at a different locus in the genome. Finally the deletion mutant *Amycolatopsis* sp. ATCC 39116 Δ*vdh* (Strain F33) was verified by sequencing of the 2.42-kb fragment; the obtained sequence showed a correct deletion of the whole coding sequence of *vdh.*

After demonstrating a beneficial impact of the *vdh* deletion on the accumulation of vanillin by *Amycolatopsis* sp. ATCC 39116 in biotransformation experiments the new markerless mutant *Amycolatopsis* sp. ATCC 39116 Δ*vdh* was analysed during fermentation. Therefore, F33 was compared to the wild type to analyse the influence of VDH_{ATCC 39116} on the catabolism of the desired product. The fermentation was performed at the 2-liter bioreactor scale. The initial volume of the starting culture was 1,000 ml. In contrast to previous studies a very simple fermentation broth, consisting of 4 g/l glucose and 4 g/l yeast extract was used. Since a significant effect of malt extract was not observable in previous growth experiments it was omitted from the basal medium to reduce the costs and to simplify the process. The fermentation was conducted in two stages. At first, the cells were grown until they reached the stationary growth phase, which was obvious due to a significant increase of the pO₂. Following the biotransformation of ferulic acid to vanillin was started by continuous feeding of the substrate. The feeding rate was adjusted, depending on the concentration of ferulic acid in the medium, which was analysed by HPLC in intervals of 1 h. Due to the toxic properties of phenolic compounds it was intended to keep the substrate concentration beneath 5 g/l but not less than 1 g/l to ensure an appropriate supply for the cells. Glucose and yeast extract were added to the feeding solution, since the supplementation was shown to positively influence the biotransformation in previous test fermentations.

The catabolism auf ferulic acid and therefore the accumulation of vanillin started after an initial phase of adaption by the wild type and F33, respectively (Fig. 2 B). During the first three hours the ferulic acid concentration increased due to a continuous supply. After the cells had adapted to the biotransformation conditions the substrate concentration decreased in both fermentations until it was kept constant between 2 and 3 g/l by adjusting the feeding rate. After 28h the feeding was stopped and the substrate concentration decreased to approximately 1 g/l during the fermentation of the wild type and the mutant. The ferulic acid catabolism was not affected by the *vdh* deletion as both processes proceeded in a comparable manner (**Figure 2A**).

However, a significant difference was observed for the accumulation of the desired product vanillin and the unintended by-product vanillic acid. The synthesis of vanillin by strain F33 was remarkably increased and resulted in an end concentration of 17.5 g/l after 23 h of biotransformation. In comparison, the wild type *Amycolatopsis* sp. ATCC 39116 formed only up to 14.1 g/l of vanillin in the same time. A further increase of the vanillin concentration was not observed in both processes, although ferulic acid was still available for the cells (**Figure 2A**). Therefore, the fermentations were stopped after 33 h.

Concerning the catabolism of vanillin, F33 showed a dramatically decreased formation of vanillic acid (0.34 g/l end concentration) while the wild type catabolized vanillin continuously, resulting in 3.2 g/l vanillic acid at the end of the fermentation. Other intermediates of the catabolism like guaiacol or protocatechuic acid and other by-products like vanillyl alcohol were only detected in trace amounts.

A total amount of 43.3 g ferulic acid was fed in both fermentations. With regard to the final vanillin concentration strain F33 accumulated the desired product with a total molar yield of 91.0%, while the wild type cells formed vanillin with a yield of only 74.8%. The results are shown in **Table 2.**

**Table 2**

| Strain improvement by metabolic engineering: Results of fermentation processes at the 2I-scale. | | | | | |
|---|---|---|---|---|---|
| **Batch** | **Ferulic acid (total amount) [g]** | **Ferulic acid (catabolized) [g]** | **Vanillin (final concentration) [g/l]** | **Vanillin (total amount) [g]** | **Molar yield (Fa → Van)** |
| **F84** | 48.1 | 46.4 | 19.3 | 34.8 | 92.0 % |
| **F33** | 43.3 | 41.1 | 17.5 | 31.4 | 91.0 % |
| **ATCC 39116** | 43.3 | 41.4 | 14.4 | 25.8 | 74.8 % |

### REVERSE TRANSCIPTASE (RT-) -PCR ANALYSIS OF ECH EXPRESSION

The fermentation experiments indicated an inefficient ferulic acid degradation and vanillin accumulation by *Amycolatopsis* sp. ATCC 39116 and mutant F33, before the cells somehow adapted to the feeding of ferulic acid within the first hours of the biotransformation phase (Fig. 2). RT-PCR analyses were conducted to figure out, if this inducing effect occurs at the gene expression level. For this, samples of the fermentation broth from the F33 and wildtype experiment were taken (i) at the end of the growth phase prior to the substrate feeding and (ii) after 7 hours of biotransformation, after the rate of vanillin synthesis had reached its maximum. The cells were harvested by centrifugation and RNA was isolated as described in materials and methods. The enzymes feruloyl-CoA synthetase (Fcs) and enoyl-CoA hydratase/aldolase (Ech), which are responsible for the catabolism of ferulic acid to vanillin in *Amycolatopsis* sp. ATCC 39116, are encoded by the respective genes *ech and fcs.* The open reading frames of both genes are located adjacently in the genome of *Amycolatopsis* sp. ATCC 39116, whereby the start codon of *fcs* (GTG) overlaps with the stop codon (TGA) of *ech.* Along with the function of the encoded enzymes, this genetic organization indicates that both genes are co-transcribed under the control of the same promoter. To provide evidence that the gene expression is induced after supplementation of ferulic acid, the transcription of *ech* was analyzed through RT-PCR. Therefore, an internal 529 bp fragment was amplified after preparation of cDNA by reverse transcription.

The RT-PCR analysis revealed a strong inducing effect of the ech-expression after supplementation of ferulic acid. Whereas for the sample taken prior to the substrate feeding only a faint band could be detected (Fig. S1, lane 1), a strong amplificate was observed for the sample, which was taken during the biotransformation phase of the fermentation (Fig. S1, lane 3). DNA contamination of the isolated RNA could be excluded in a control PCR experiment that was conducted with RNA as template without the previous reverse transcription reaction (Fig. S1, lane 2 and 4).

Additional RT-PCR analysis revealed that *ech* and *fcs* are transcribed as an operon in a single mRNA, since an overlapping 624 bp fragment from both genes could be amplified (Fig. 3, lane 3, ,5, 7) using a forward primer binding in *ech* and a reverse primer binding in *fcs*. The inducing effect was also obvious in this experiment, as a strong amplificate was only observable for the sample, which was taken during the biotransformation phase of the fermentation (Fig. 3, lane 3).

### CONSTRUCTION AND APPLICATION OF AN OVEREXPRESSION MUTANT OF AMYCOLATOPSIS SP. ATCC 39116 ΔVDH

To further improve the conversion of ferulic acid to vanillin, an overexpression mutant for *ech* and *fcs* was generated. For this purpose, additional copies of both genes were integrated in the genome of F33 under the control of the strong constitutive promoter *permE*.* For the integration, the former *vdh*-locus was chosen, since neither the deletion of *vdh* nor the replacement of the gene with a kanamycin resistance cassette led to negative polar effects. Furthermore, the suicide plasmid p6suigusA::Δ*vdh* could be used for the targeted integration via homologous recombination.

First, both genes were amplified together from the genome of *Amycolatopsis* sp. ATCC 39116 and cloned in the *Amycolatopsis* expression vector p6permE collinear to *permE*.* In a second step the genes were amplified together with the promoter, and the resulting fragment was cloned blunt in the *vdh* deletion construct of p6suigusA::Δ*vdh*. The resulting plasmid p6suiΔ*vdh*::*permE**::*echfcs* was used to transform strain F33. Although apramycin resistant clones were obtained, we faced the same problem of illegitimate integration of the suicide plasmid as mentioned above. Because of this, the integration of the suicide plasmid at the *vdh*-locus was analysed by amplification of the genomic area first. A correct integration of a 15.1-kb fragment, which comprised the flanking regions of *vdh* and the suicide plasmid, could be identified. After verifying the desired homologous recombination, the clone was further cultivated to achieve a second recombination event as described for the *vdh* deletion. The accurate integration of the overexpression construct could be achieved in one mutant and was confirmed by diagnostic PCR (**Figure 1**) and also by sequencing of the PCR product. The new mutant *Amycolatopsis* sp. ATCC 39116 Δ*vdh::permE*::echfcs* (strain F84) should combine the beneficial impact of the *vdh* knockout with an increased ferulic acid catabolism rate.

For a validation of the new expected phenotype, strain F84 was cultivated in fermentation experiments at the 2-liter scale, as well. In order to analyse the impact of the overexpression of *ech* and *fcs* on the catabolism of ferulic acid and the accumulation of vanillin, the wild type *Amycolatopsis* sp. ATCC 39116 and the generated mutants F33 and F84 were cultivated in parallel experiments under the same conditions as described above. The effect of the enhanced expression of *ech* and *fcs* was obvious directly after the biotransformation phase was started by feeding the ferulic acid solution (**Figure 2A**). While the wild type and strain F33 exhibited a lag phase of about 3 h before the catabolism of ferulic acid increased and reached its maximum, conversion of ferulic acid to vanillin started in F84 directly after supplementation with improved performance. Although ferulic acid was fed at a higher rate (up to 3.5 g/h) in comparison to the wild type and F33 (up to 2.6 g/h), the concentration of ferulic acid was continuously lower during the entire time course of the fermentation. The feeding strategy was adapted due to the higher conversion rate and in total 4.9 g more substrate could be fed in an even shorter time period of only 18 hours **(****Figure 2A****).**

The enhancing effect was also evident for the accumulation of vanillin, which was synthesized by F84 without the previously observed adaption phase in the first hours (**Figure 2B****).** This resulted in a maximal production rate of 1.6 g/l/h and a vanillin concentration of 8.1 g/l after already 6 h of the biotransformation phase (wild type: 7.8 g/l, 10 h). As aspired, the increased metabolism of ferulic acid to vanillin led to a higher final concentration of 19.3 g/l with a total molar vanillin yield of 92.0 % with regard to the added substrate (see also **Table 2)** Concerning the consumed ferulic acid, the molar yield was even higher (96.2%), given that 0.91 g/l of the substrate remained in the medium at the end, when no further synthesis of vanillin occurred.

Concerning the catabolism of vanillin, the new mutant F84 showed a comparable behaviour like F33. This was expected due to the deletion of *vdh* in both strains. Interestingly, F84 showed an even slightly decreased formation of vanillic acid, although more vanillin was generated during the cultivation of this mutant (**Figure 2C**).

### VANILLIN TOXICITY TEST

The fermentation experiments revealed an unknown limitation of the production of vanillin, although ferulic acid was still available for the cells (**Figure 2C**). It is known that vanillin and other phenolic compounds exhibit antimicrobial properties. Although *Amycolatopsis* sp. ATCC 39116 is known to tolerate higher concentrations of vanillin than all other tested organisms, the fermentation parameters, which were recorded, clearly indicated that the cell metabolism is affected also in this bacterium by increasing concentrations of the desired product. The consumption of dissolved oxygen as well as the cell density was evidently decreasing when the process was approaching the final stage.

To elucidate the effect of vanillin on the viability of *Amycolatopsis* sp. ATCC 39116, the cells were cultivated in shake flasks until they reached the stationary growth phase as also done in the fermentation experiments. Subsequently from this culture six aliquots were prepared, and different concentrations of vanillin in the range from 2 g/l to 10 g/l were added. Samples where then withdrawn at given time points from the cultures, diluted and plated on agar plates at given time points to count colony forming units. The results represent the mean of four independent replicates.

The experiments clearly showed the antimicrobial effect of vanillin (**Figure 4**). While a concentration of up to 4 g/l is tolerated by the cells after an initial decrease of the viable cell concentration, 7 g/l led to complete cell death within 6 hours. Moreover, a vanillin concentration of 10 g/l led to 90% cell death within only one hour; no viable cells could be detected after only 3 hours of incubation. Since a higher vanillin concentration could be detected in fermentation experiments, it was also tested if an adaption of the cells to the product might result in a better tolerance.

For this, 2 g/l vanillin was added to a culture, as it was shown to be tolerated by the cells in the initial experiment (**Figure 4**). Subsequently, the concentration was raised to 10 g/l after one or three hours of cultivation, respectively. However, the results of this experiment showed that there was no adaption of the cells that might lead to a higher tolerance to vanillin. The exposure to 10 g/l vanillin again led to up to 90% cell death in the following hour.

## Claims

1. A culture of Amycolatopsis sp. ATCC 39116 - mutant *Amycolatopsis* sp. ATCC 39116 Δ*vdh* which is F33 or mutant *Amycolatopsis* sp. ATCC 39116 Δ*vdh::permE*::echfcs* which is F84 and converts ferulic acid into vanillin, **characterized in that**,
the mutant F33 is obtained by
(a1) digesting *E. coli* - Amycolatopsis shuttle vector p6apra with *Nae*I/*SacI*I to eliminate the essential part of the origin of replication for Amycolatopsis and to obtain the resulting fragment (3575 bp) containing *oriV* for replication in *E. coli,* the apramycin resistance gene and several unique restriction sites for cloning;
(a2) religating the fragment and transforming *E. coli* with the resulting plasmid p6sui;
(a3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(a4) deleting of the vdh gene by homologous recombination, wherein the flanking regions upstream and downstream of *vdh* in the genome of *Amycolatopsis* sp. ATCC 39116 are amplified using the following oligonucleotides:
upstream region:
*vdh*LF*_*for3 (5'-TCGTACTTCGCGGTGATCTCGTGG-3')
and
*vdh*LF_rev_RF (5'-GCGCATCAGGAGTGACACGCGCGGCGGGGGCG-3');
downstream region:
*vdh*RF_for_LF (5'-CGCCCCCGCCGCGCGTGTCACTCCTGATGCGC-3') and
*vdh*RF_rev3 (5'-TTGCAGTCCTTTGTAGAGCGACACG-3');
(a5) purifiying the resulting amplificates and combining them in a subsequent fusion-PCR using primers *vdh*LF_for3 and *vdh*RF_rev3;
(a6) cloning the resulting fragment Δ*vdh* including the upstream and downstream regions of *vdh* into the *Eco*RV-site of the suicide plasmid p6suigusA;
(a7) isolating the resulting plasmid p6suigusA::Δ*vdh* from *E. coli* and transferring the vector to *Amycolatopsis* sp. ATCC 39116; and
(a8) selecting transformants of *Amycolatopsis* sp. ATCC 39116 with regard to correct genotype and cultivating them to obtain a second recombinant event;
and the mutant F84 is obtained by
(b1) digesting *E. coli* - *Amycolatopsis* shuttle vector p6apra with *Nae*I/*Sac*II to eliminate the essential part of the origin of replication for Amycolatopsis and to obtain the resulting fragment (3575 bp) containing *oriV* for replication in *E. coli,* the apramycin resistance gene and several unique restriction sites for cloning;
(b2) religating the fragment and transforming *E. coli* with the resulting plasmid p6sui;
(b3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(b4) amplifying the genes ech and fcs together from genomic DNA of *Amycolatopsis* sp. ATCC 39116 using oligonucleotides
*ech_*for*_Sac*I (5'-AAAAGAGCTCTAAGGAGGTGACAACTGCTGGC CGCGCTCG-3') and
*fcs*_rev_*X*baI (5'-AAAATCTAGACCACAGAGTAGCCGCAGCGGG-3');
(b5) digesting the PCR product with SacI and XbaI and subsequently cloning it into the *Sac*I/*Xba*I-linearized pBluescript SK⁻.
(b6) transferring the resulting plasmid *pSK::echfcs* to *E. coli* Mach-1 T;
(b7) digesting *pSK::echfcs* with *Eco*ICRI and *Hin*dIII*,* and ligating the resulting fragment, containing both genes, with the *Fsp*AI/*Hin*dIII-linearized expression vector p6permE, collinear to *permE*;*
(b8) amplifying a fragment, comprising the promoter *permE** and the genes *ech* and *fcs* from the resulting plasmid p6permE::*echfcs* using oligonucleotides
p6apraMCS_for (5'-GCGAGCACCGGAGGCAGG-3') and
p6apraMCS_rev (5'-CATTCATCCGGGGTCAGCAC-3')
(b9) cloning the blunt PCR product in the *Hinc*II-linearized suicide plasmid p6suigusA::Δ*vdh*, to achieve an integration at the Δ*vdh-*locus;
(b10) transferring the resulting plasmid p6suiΔ*vdh*::permE*::*echfcs* to *Amycolatopsis* sp. ATCC 39116 Δ*vdh.*
(b11) selecting transformants of *Amycolatopsis* sp. ATCC 39116 with regard to correct genotype and cultivating them to obtain a second recombinant event.

2. A process for preparing vanillin comprising subjecting ferulic acid to Amycolatopsis sp. ATCC 39116 - mutant *Amycolatopsis* sp. ATCC 39116 Δ*vdh* which is F33 as defined in Claim 1 or mutant *Amycolatopsis* sp. ATCC 39116 Δ*vdh::permE*::echfcs* which is F84 as defined in Claim 1 which converts ferulic acid into vanillin, for a period of time sufficient to convert said ferulic acid to vanillin, and recovering the vanillin thus formed.

3. The process of Claim 2, wherein said ferulic acid is natural ferulic acid.

4. The process of Claim 2, wherein said microorganism is used to convert ferulic acid to vanillin and the microorganism is contained in a medium which comprises a carbon source.

5. The process of Claim 4, wherein the carbon source is selected from the group consisting of sugars, sugar alcohols, organic acids and complex mixtures.

6. The process of Claim 4, wherein said medium further comprises a nitrogen source.

7. A process for obtaining the *Amycolatopsis* sp. ATCC 39116 Δ*vdh* mutant of *Amycolatopsis* sp. ATCC 39116, encompassing the following steps:
(a1) digesting *E. coli* - Amycolatopsis shuttle vector p6apra with *Nae*I/*SacI*I to eliminate the essential part of the origin of replication for Amycolatopsis and to obtain the resulting fragment (3575 bp) containing *oriV* for replication in *E. coli,* the apramycin resistance gene and several unique restriction sites for cloning;
(a2) religating the fragment and transforming *E. coli* with the resulting plasmid p6sui;
(a3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(a4) deleting of the vdh gene by homologous recombination, wherein the flanking regions upstream and downstream of *vdh* in the genome of *Amycolatopsis* sp. ATCC 39116 are amplified using the following oligonucleotides:
upstream region:
*vdh*LF*_*for3 (5'-TCGTACTTCGCGGTGATCTCGTGG-3')
and
*vdh*LF_rev_RF (5'-GCGCATCAGGAGTGACACGCGCGGCGGGGGCG-3');
downstream region:
*vdh*RF_for_LF (5'-CGCCCCCGCCGCGCGTGTCACTCCTGATGCGC-3') and
vdhRF_rev3 (5'-TTGCAGTCCTTTGTAGAGCGACACG-3');
(a5) purifiying the resulting amplificates and combining them in a subsequent fusion-PCR using primers *vdh*LF_for3 and *vdh*RF_rev3;
(a6) cloning the resulting fragment Δ*vdh* including the upstream and downstream regions of *vdh* into the *Eco*RV-site of the suicide plasmid p6suigusA;
(a7) isolating the resulting plasmid p6suigusA::Δ*vdh* from *E. coli* and transferring the vector to *Amycolatopsis* sp. ATCC 39116; and
(a8) selecting transformants of *Amycolatopsis* sp. ATCC 39116 with regard to correct genotype and cultivating them to obtain a second recombinant event.

8. A process for obtaining the *Amycolatopsis* sp. ATCC 39116 Δ*vdh::permE*::echfcs* mutant of Amycolatopsis sp. ATCC 39116, encompassing the following steps:
(b1) digesting *E. coli* - *Amycolatopsis* shuttle vector p6apra with *Nae*I/*Sac*II to eliminate the essential part of the origin of replication for Amycolatopsis and to obtain the resulting fragment (3575 bp) containing *oriV* for replication in *E. coli,* the apramycin resistance gene and several unique restriction sites for cloning;
(b2) religating the fragment and transforming *E. coli* with the resulting plasmid p6sui;
(b3) isolating the glucuronidase gene *gusA* as *Spe*I/*Eco*RV fragment from pSETGUS and cloning into the *Nhe*I/*Dra*I-linearized suicide plasmid p6sui to obtain p6suigusA;
(b4) amplifying the genes ech and fcs together from genomic DNA of *Amycolatopsis* sp. ATCC 39116 using oligonucleotides
*ech_*for*_Sac*I (5'-AAAAGAGCTCTAAGGAGGTGACAACTGCTGGC CGCGCTCG-3') and
*fcs*_rev_*X*baI (5'-AAAATCTAGACCACAGAGTAGCCGCAGCGGG-3');
(b5) digesting the PCR product with Sacl and XbaI and subsequently cloning it into the *Sac*I/*Xba*I-linearized pBluescript SK⁻.
(b6) transferring the resulting plasmid *pSK::echfcs* to *E. coli* Mach-1 T;
(b7) digesting *pSK::echfcs* with *Eco*ICRI and *Hind*III*,* and ligating the resulting fragment, containing both genes, with the *Fsp*AI/HindIII-linearized expression vector p6permE, collinear to *permE*;*
(b8) amplifying a fragment, comprising the promoter *permE** and the genes *ech* and *fcs* from the resulting plasmid p6permE::*echfcs* using oligonucleotides
p6apraMCS_for (5'-GCGAGCACCGGAGGCAGG-3') and
p6apraMCS_rev (5'-CATTCATCCGGGGTCAGCAC-3')
(b9) cloning the blunt PCR product in the *Hinc*II-linearized suicide plasmid p6suigusA::Δ*vdh*, to achieve an integration at the Δ*vdh-*locus;
(b10) transferring the resulting plasmid p6suiΔ*vdh*::permE*::*echfcs* to *Amycolatopsis* sp. ATCC 39116 Δ*vdh.*
(b11) selecting transformants of *Amycolatopsis* sp. ATCC 39116 with regard to correct genotype and cultivating them to obtain a second recombinant event.

## Patentansprüche

1. Eine Kultur von Amycolatopsis sp. ATC 39116 - Mutante *Amycolatopsis sp.* ATCC 39116 Δ*vdh,* welche F33 ist oder Mutante *Amycolatopsis sp.* ATCC 39166 Δ*vdh::permE*::echfcs,* welche F 84 ist und welche Ferulasäure in Vanillin umwandelt, **dadurch gekennzeichnet, dass**
die Mutante F33 erhalten wird durch
(a1) Schneiden des *E.coli* - Amycolatopsis Shuttle Vektors p6apra mit *NaeI*/*SacII* um den wichtigen Teil des Replikationsursprungs für Amycolatopsis zu eliminieren und um das resultierende Fragment (3575 bp) enhtaltend oriVfür die Replikation in E.coli, das Apramycin Resistenzgen und verschiedene einzigartige restriction sites für die Klonierung zu erhalten;
(a2) Religieren des Fragments und Transformation des resultierenden Plasmids p6sui in *E.coli;*
(a3) Isolierung der Glucuronidase Gene *gusA* als *SpeI*/*EcoRV* Fragment von pSETGUS und Klonierung in das *NheI*/*DraI*-linearisierte Suicide Plasimd p6sui um p6suigusA zu erhalten;
(a4) Ausschalten des vdh Gens durch homologe Rekombination, wobei die flankierenden Regionen von *vdh* im Genom von *Amycolatopsis sp.* ATCC 39116 aufwärts und abwärst amplifiziert werden, mit den folgenden Oligonukleotiden:
Region aufwärts:
*vdh*LF_for3 (5'- TCGTACTTCGCGGTGATCTCGTGG-3')
und
*vdh*LF_rev_RF (5'-GCGCATCAGGAGTGACACGCGCGGCGGGGGCG-3');
Region abwärts:
*vdh*RF_for_LF (5'-CGCCCCCGCCGCGCGTGTCACTCCTGATGCGC-3') und
*vdh*RF_rev3 (5'-TTGCAGTCCTTTGTAGAGCGACACG-3');
(a5) Aufreinigung der resultierenden Amplifikate und Kombinieren dieser in einer nachfolgenden fusion PCR mit den Primern *vdhLF_*for3 und *vdf*RF_rev3;
(a6) Klonierung des resultierenden Fragments Δ*vdh* enthaltend die aufwärts und abwärts Regionen von vdh in der EcoRV-Seite des Suicide Plasmids p6suigusA;
(a7) Isolierung des resultierenden Plasmids p6suigusA::Δ*vdh* von *E.coli* und Transferieren des Vektors zu Amycolatopsis sp. ATCC 39116; und
(a8) Selektion der Tranformanten von Amycolatopsis sp. ATCC 39166 in Bezug auf den richtigen Genotyp und Kultivierung dieser um ein zweites Rekombinationsereignis zu erhalten;
und die Mutante F84 erhalten wird durch
(b1) Schneiden des *E.coli* - Amycolatopsis Shuttle Vektors p6apra mit NaeI/*Sac*II um den wichtigen Teil des Replikationsursprungs für Amycolatopsis zu eliminieren und um das resultierende Fragment (3575 bp) enhtaltend *oriV* für die Replikation in *E.coli,* das Apramycin Resistenzgen und verschiedene einzigartige restriction sites für die Klonierung zu erhalten;
(b2) Religieren des Fragments und Transformation des resultierenden Plasmids p6sui in *E.coli;*
(b3) Isolierung der Glucuronidase Gene *gusA* als *Spe*I/*Eco*RV Fragment von pSETGUS und Klonierung in das *Nhe*I/*Dra*I-linearisierte Suicide Plasimd p6sui um p6suigusA zu erhalten;
(b4) Amplifizieren der Gene ech und fcs zusammen von der genomischen DNA von Amycolatopsis sp. ATCC 39116 unter Verwendung der Oligonukleotide
*ech*_for_SacI (5'-AAAAGAGCTCTAAGGAGGTGACAACTGCTGGC CGCGCTCG-3') und
*fcs*_rev_XbaI (5'-AAAATCTAGACCACAGAGTAGCCGCAGCGGG-3');
(b5) Schneiden des PCR Produkts mit *Sac*I und *Xba*I und nachfolgendes Klinieren in den *SacI*/*Xba*I-linearisierten pBluescript SK;
(b6) Transferieren des resultierenden Plasmids pSK::echfcs in *E.coli* Mach-1T;
(b7) Schneiden von pSK ::echfs mit *Eco*/CRI und *Hind*III und Ligieren des resultierenden Fragments, enthaltend beide Gene, mit *Fsp*AI/*Hind*III-I inearisiertem Expressionsvektor p6permE, koliniear zu *permE**;
(b8) Amplifizieren eines Fragments, umfassend den Promotor *permE** und die Gene ech und fcs aus dem resultierenden Plasmid p6permE::*echfs* unter Verwendung der Oligonukleotide
p6apraMCS_for (5'-GCGAGCACCGGAGGCAGG-3') und
p6apraMCS_rev(5'-CATTCATCCGGGGTCAGCAC-3');
(b9) Klonierung des geraden PCR Produktes in das HincII-linearisierte Suicide Plasmid p6suigusA::Δ*vdh*, um eine Integration a, *Δvdh*-Locus zu erreichen;
(b10) Transferieren des resultierenden Plasmids p6suiΔvdh::permE*::*echfcs* in Amycolatopsis sp- ATCC 39116 *Δvdh;*
(b11) Selektion der Transformanten von Amycolatopsis sp- ATCC 39116 in Bezug auf den richtigen Genotyp und Kultivierung dieser um ein zweites Rekombinationsereignis zu erhalten.

2. Ein Verfahren zur Herstellung von Vanillin umfassend das Aussetzen von Ferulasäure dem Amycolatopsis sp. ATCC 39166 - Mutante *Amycolatopsis sp.* ATCC 39116 *Δvdh,* welche F33, wie in Anspruch 1 definiert, ist oder Mutante *Amycolatopsis sp.* ATCC 39166 *Δvdh::permE*::echfcs,* welche F 84, wie in Anspruch 1 definiert ist, welche Ferulasäure in Vanillin umwandeln, für eine gewisse Zeit, die ausreichend ist die Ferulasäure in Vanillin umzuwandeln, und die Wiedergewinnung des so gebildeteten Vanillins.

3. Das Verfahren nach Anspruch 2, wobei die Ferulasäure natürliche Ferulasäure ist.

4. Das Verfahren nach Anspruch 2, wobei der Mikroorganismus verwendet wird um Ferulasäure in Vanillin umzuwandeln und der Mikroorganismus in einem Medium enthalten ist, welches eine Kohlenstoffquelle enthält.

5. Das Verfahren nach Anspruch 4, wobei die Kohlenstoffquelle ausgewählt ist aus der Gruppe bestehend aus Zuckern, Zuckeralkoholen, organischen Säuren und komplexen Mischungen.

6. Das Verfahren nach Anspruch 4, wobei das Medium weiterh eine Stickstoffquelle umfasst.

7. Ein Verfahren zur Erhaltung der *Amycolatopsis sp.* ATCC 39166 *Δvdh* Mutante von *Amycolatopsis* sp. ATCC 39116, aufweisend die folgenden Schritte:
(a1) Schneiden des *E.coli* - Amycolatopsis Shuttle Vektors p6apra mit *NaeI*/*SacII* um den wichtigen Teil des Replikationsursprungs für Amycolatopsis zu eliminieren und um das resultierende Fragment (3575 bp) enhtaltend *oriV* für die Replikation in *E.coli,* das Apramycin Resistenzgen und verschiedene einzigartige restriction sites für die Klonierung zu erhalten;
(a2) Religieren des Fragments und Transformation des resultierenden Plasmids p6sui in *E.coli;*
(a3) Isolierung der Glucuronidase Gene *gusA* als *Spe*I/*Eco*RV Fragment von pSETGUS und Klonierung in das *NheI*/*DraI*-linearisierte Suicide Plasimd p6sui um p6suigusA zu erhalten;
(a4) Ausschalten des vdh Gens durch homologe Rekombination, wobei die flankierenden Regionen von *vdh* im Genom von *Amycolatopsis sp.* ATCC 39116 aufwärts und abwärst amplifiziert werden, mit den folgenden Oligonukleotiden:
Region aufwärts:
*vdh*LF*_*for3 (5'- TCGTACTTCGCGGTGATCTCGTGG-3')
und
*vdh*LF_rev_RF (5'-GCGCATCAGGAGTGACACGCGCGGCGGGGGCG-3');
Region abwärts:
*vdh*RF_for_LF (5'-CGCCCCCGCCGCGCGTGTCACTCCTGATGCGC-3') und
*vdh*RF_rev3 (5'-TTGCAGTCCTTTGTAGAGCGACACG-3');
(a5) Aufreinigung der resultierenden Amplifikate und Kombinieren dieser in einer nachfolgenden fusion PCR mit den Primern *vdh*LF_for3 und *vdh*RF_rev3;
(a6) Klonierung des resultierenden Fragments Δ*vdh* enthaltend die aufwärts und abwärts Regionen von vdh in der EcoRV-Seite des Suicide Plasmids p6suigusA;
(a7) Isolierung des resultierenden Plasmids p6suigusA::*Δvdh* von *E.coli* und Transferieren des Vektors zu Amycolatopsis sp. ATCC 39116; und
(a8) Selektion der Tranformanten von Amycolatopsis sp. ATCC 39166 in Bezug auf den richtigen Genotyp und Kultivierung dieser um ein zweites Rekombinationsereignis zu erhalten.

8. Ein Verfahren zur Erhaltung der *Amycolatopsis sp.* ATCC 39166 *Δvdh ::permE* ::echfs* Mutante von *Amycolatopsis* sp. ATCC 39116, aufweisend die folgenden Schritte:
(b1) Schneiden des *E.coli* - Amycolatopsis Shuttle Vektors p6apra mit *Nae*I/*Sac*II um den wichtigen Teil des Replikationsursprungs für Amycolatopsis zu eliminieren und um das resultierende Fragment (3575 bp) enhtaltend *oriV* für die Replikation in *E.coli,* das Apramycin Resistenzgen und verschiedene einzigartige restriction sites für die Klonierung zu erhalten;
(b2) Religieren des Fragments und Transformation des resultierenden Plasmids p6sui in *E.coli;*
(b3) Isolierung der Glucuronidase Gene *gusA* als *Spe*I/*Eco*RV Fragment von pSETGUS und Klonierung in das *Nhe*I/*Dra*I-linearisierte Suicide Plasimd p6sui um p6suigusA zu erhalten;
(b4) Amplifizieren der Gene ech und fcs zusammen von der genomischen DNA von Amycolatopsis sp. ATCC 39116 unter Verwendung der Oligonukleotide
*ech_*for_SacI (5'-AAAAGAGCTCTAAGGAGGTGACAACTGCTGGC CGCGCTCG-3') und
*fcs*_rev_XbaI (5'-AAAATCTAGACCACAGAGTAGCCGCAGCGGG-3');
(b5) Schneiden des PCR Produkts mit *Sac*I und *Xba*I und nachfolgendes Klinieren in den *SacI*/*Xba*I-linearisierten pBluescript SK;
(b6) Transferieren des resultierenden Plasmids pSK::echfcs in *E.coli* Mach-1 T;
(b7) Schneiden von pSK ::echfs mit Eco*I*CRI und *Hind*III und Ligieren des resultierenden Fragments, enthaltend beide Gene, mit *Fsp*AI/*Hind*III-I inearisiertem Expressionsvektor p6permE, koliniear zu *permE*;*
(b8) Amplifizieren eines Fragments, umfassend den Promotor *permE** und die Gene ech und fcs aus dem resultierenden Plasmid p6permE::*echfs* unter Verwendung der Oligonukleotide
p6apraMCS_for (5'-GCGAGCACCGGAGGCAGG-3') und
p6apraMCS_rev(5'-CATTCATCCGGGGTCAGCAC-3');
(b9) Klonierung des geraden PCR Produktes in das HincII-linearisierte Suicide Plasmid p6suigusA::Δ*vdh*, um eine Integration a, *Δvdh-*Locus zu erreichen;
(b10) Transferieren des resultierenden Plasmids p6suiΔvdh::permE*::echfcs in Amycolatopsis sp- ATCC 39116 *Δvdh;*
(b11) Selektion der Transformanten von Amycolatopsis sp- ATCC 39116 in Bezug auf den richtigen Genotyp und Kultivierung dieser um ein zweites Rekombinationsereignis zu erhalten.

## Revendications

1. Culture d'Amycolatopsis sp. ATCC 39116 - un Δ*vdh* mutant d'Amycolatopsis sp. ATCC 39116 qui est le F33 ou un Δvdh::permE*::echfcs mutant d'Amycolatopsis sp. ATCC 39116 qui est le F84 et qui convertit l'acide férulique en vanilline, **caractérisée en ce que**
le mutant F33 est obtenu en
(a1) digérant le vecteur navette p6apra d'E. coli - Amycolatopsis avec Nael/Sacll pour éliminer la partie essentielle de l'origine de réplication pour Amycolatopsis et pour obtenir le fragment résultant (3575 pb) contenant l'oriV pour une réplication dans E. coli, le gène de résistance à l'apramycine et plusieurs sites de restriction uniques for un clonage ;
(a2) ligaturant à nouveau le fragment et en transformant E. coli avec le plasmide résultant p6sui ;
(a3) isolant le gène gusA de la glucuronidase, sous forme d'un fragment Spel/EcoRV provenant de pSETGUS, et par un clonage dans le plasmide suicide p6sui linéarisé avec Nhel/Dral pour obtenir le p6suigusA;
(a4) supprimant le gène vdh par une recombinaison homologue, dans laquelle les régions flanquantes en amont et en aval du vdh dans le génome d'Amycolatopsis sp. ATCC 39116 sont amplifiées en utilisant les oligonucléotides suivants :
région en amont :
vdhLF_for3 (5'-TCGTACTTCGCGGTGATCTCGTGG-3')
et
vdhLF_rev_RF (5'-GCGCATCAGGAGTGACACGCGCGGCGGGGGCG-3') ;
région en aval:
vdhRF_for_LF (5'-CGCCCCCGCCGCGCGTGTCACTCCTGATGCGC-3') et
vdhRF_rev3 (5'-TTGCAGTCCTTTGTAGAGCGACACG-3');
(a5) purifiant les produits d'amplification résultants et en les combinant dans une ACP de fusion ultérieure, en utilisant les amorces vdhLF_for3 et vdhRF_rev3 ;
(a6) clonant le fragment Δvdh résultant, incluant les régions en amont et en aval du vdh, dans le site EcoRV du plasmide suicide p6suigusA ;
(a7) isolant le plasmide p6suigusA::Δvdh résultant à partir d'E. coli et en transférant le vecteur vers Amycolatopsis sp. ATCC 39116 ; et en
(a8) sélectionnant des transformants d'Amycolatopsis sp. ATCC 39116 en fonction du génotype correct et en les cultivant pour obtenir un deuxième événement recombinant;
et le mutant F84 est obtenu en
(b1) digérant le vecteur navette p6apra d'E. coli - Amycolatopsis avec Nael/Sacll pour éliminer la partie essentielle de l'origine de réplication pour Amycolatopsis et pour obtenir le fragment résultant (3575 pb) contenant l'oriV pour une réplication dans E. coli, le gène de résistance à l'apramycine et plusieurs sites de restriction uniques for un clonage ;
(b2) ligaturant à nouveau le fragment et en transformant E. coli avec le plasmide résultant p6sui ;
(b3) isolant le gène gusA de la glucuronidase, sous forme d'un fragment Spel/EcoRV provenant de pSETGUS, et par un clonage dans le plasmide suicide p6sui linéarisé avec Nhel/Dral pour obtenir le p6suigusA;
(b4) amplifiant les gènes ech et fcs ensemble à partir d'ADN génomique d'Amycolatopsis sp. ATCC 39116 en utilisant les oligonucléotides
ech_for_SacI (5'-AAAAGAGCTCTAAGGAGGTGACAACTGCTGGC CGCGCTCG-3')
et
fcs_rev_XbaI (5'-AAAATCTAGACCACAGAGTAGCCGCAGCGGG-3') ;
(b5) digérant le produit de l'ACP avec SacI et XbaI et en le clonant ultérieurement dans le pBluescript SK- linéarisé avec SacI/XbaI ;
(b6) transférant le plasmide pSK::echfcs résultant vers E. coli Match-1 T;
(b7) digérant le pSK::echfcs avec EcoICRI et Hindlll, et en ligaturant le fragment résultant, contenant les deux gènes, avec le vecteur d'expression p6permE linéarisé avec FspAI/HindIII, colinéaire à *permE**;
(b8) amplifiant un fragment, qui comprend le promoteur *permE** ainsi que les gènes ech et fcs, à partir du plasmide p6permE::echfcs résultant en utilisant les oligonucléotides
p6apraMCS_for (5'-GCGAGCACCGGAGGCAGG-3') et
p6apraMCS_rev(5'-CATTCATCCGGGGTCAGCAC-3')
(b9) clonant le produit de l'ACP à extrémités franches dans le plasmide suicide p6suigusA::Δvdh linéarisé avec HincII, pour atteindre une intégration au niveau du locus du Δvdh ;
(b10) transférant le plasmide p6suiΔvdh::permE*::echfcs résultant vers l'Δvdh d'Amycolatopsis sp. ATCC 39116 ;
(b11) sélectionnant des transformants d'Amycolatopsis sp. ATCC 39116 en fonction du génotype correct et en les cultivant pour obtenir un deuxième événement recombinant.

2. Processus destiné à préparer de la vanilline, comprenant les étapes consistant à soumettre de l'acide férulique à Amycolatopsis sp. ATCC 39116 - un Δvdh mutant d'Amycolatopsis sp. ATCC 39116 qui est le F33, tel que défini dans la revendication 1, ou un Δvdh::permE*::echfcs mutant d'Amycolatopsis sp. ATCC 39116 qui est le F84, tel que défini dans la revendication 1, qui convertit l'acide férulique en vanilline, pendant une période suffisante pour convertir ledit acide férulique en vanilline, et récupérer la vanilline ainsi formée.

3. Processus de la revendication 2, dans lequel ledit acide férulique est de l'acide férulique naturel.

4. Processus de la revendication 2, dans lequel ledit microorganisme est utilisé pour convertir l'acide férulique en vanilline et le microorganisme est contenu dans un milieu qui comprend une source de carbone.

5. Processus de la revendication 4, dans lequel la source de carbone est choisie dans le groupe constitué par des sucres, alcools de sucres, acides organiques et mélanges complexes.

6. Processus de la revendication 4, dans lequel ledit milieu comprend en outre une source d'azote.

7. Processus destiné à obtenir le mutant Δvdh d'Amycolatopsis sp. ATCC 39116 d'Amycolatopsis sp. ATCC 39116, englobant les étapes suivantes :
(a1) digérer le vecteur navette p6apra d'E. coli - Amycolatopsis avec Nael/Sacll pour éliminer la partie essentielle de l'origine de réplication pour Amycolatopsis et pour obtenir le fragment résultant (3575 pb) contenant l'oriV pour une réplication dans E. coli, le gène de résistance à l'apramycine et plusieurs sites de restriction uniques for un clonage ;
(a2) ligaturer à nouveau le fragment et transformer E. coli avec le plasmide p6sui résultant ;
(a3) isoler le gène gusA de la glucuronidase, sous forme d'un fragment Spel/EcoRV provenant de pSETGUS, et réaliser un clonage dans le plasmide suicide p6sui linéarisé avec Nhel/Dral pour obtenir le p6suigusA;
(a4) supprimer le gène vdh par une recombinaison homologue, dans laquelle les régions flanquantes en amont et en aval du vdh dans le génome d'Amycolatopsis sp. ATCC 39116 sont amplifiées en utilisant les oligonucléotides suivants :
région en amont :
vdhLF_for3 (5'-TCGTACTTCGCGGTGATCTCGTGG-3')
et
vdhLF_rev_RF (5'-GCGCATCAGGAGTGACACGCGCGGCGGGGGCG-3') ;
région en aval :
vdhRF_for_LF (5'-CGCCCCCGCCGCGCGTGTCACTCCTGATGCGC-3') et
vdhRF_rev3 (5'-TTGCAGTCCTTTGTAGAGCGACACG-3');
(a5) purifier les produits d'amplification résultants et les combiner dans une ACP de fusion ultérieure, en utilisant les amorces vdhLF_for3 et vdhRF_rev3 ;
(a6) cloner le fragment Δvdh résultant, incluant les régions en amont et en aval du vdh, dans le site EcoRV du plasmide suicide p6suigusA ;
(a7) isoler le plasmide p6suigusA::Δvdh résultant à partir d'E. coli et transférer le vecteur vers Amycolatopsis sp. ATCC 39116 ; et
(a8) sélectionner des transformants d'Amycolatopsis sp. ATCC 39116 en fonction du génotype correct et les cultiver pour obtenir un deuxième événement recombinant.

8. Processus destiné à obtenir le mutant Δvdh::permE*::echfcs d'Amycolatopsis sp. ATCC 39116 d'Amycolatopsis sp. ATCC 39116, englobant les étapes suivantes :
(b1) digérer le vecteur navette p6apra d'E. coli - Amycolatopsis avec Nael/Sacll pour éliminer la partie essentielle de l'origine de réplication pour Amycolatopsis et pour obtenir le fragment résultant (3575 pb) contenant l'oriV pour une réplication dans E. coli, le gène de résistance à l'apramycine et plusieurs sites de restriction uniques for un clonage ;
(b2) ligaturer à nouveau le fragment et transformer E. coli avec le plasmide p6sui résultant ;
(b3) isoler le gène gusA de la glucuronidase, sous forme d'un fragment SpeI/EcoRV provenant de pSETGUS, et réaliser un clonage dans le plasmide suicide p6sui linéarisé avec NheI/DraI pour obtenir le p6suigusA ;
(b4) amplifier les gènes ech et fcs ensemble à partir d'ADN génomique d'Amycolatopsis sp. ATCC 39116 en utilisant les oligonucléotides
ech_for_SacI (5'-AAAAGAGCTCTAAGGAGGTGACAACTGCTGGC CGCGCTCG-3')
et
fcs_rev_Xbal (5'-AAAATCTAGACCACAGAGTAGCCGCAGCGGG-3') ;
(b5) digérer le produit de l'ACP avec SacI et XbaI et le cloner ultérieurement dans le pBluescript SK- linéarisé avec SacI/XbaI ;
(b6) transférer le plasmide pSK::echfcs résultant vers E. coli Match-1 T;
(b7) digérer le pSK::echfcs avec EcoICRI et Hindlll, et ligaturer le fragment résultant, contenant les deux gènes, avec le vecteur d'expression p6permE linéarisé avec FspAI/HindIII, colinéaire à *permE** ;
(b8) amplifier un fragment, qui comprend le promoteur permE* ainsi que les gènes ech et fcs, à partir du plasmide p6permE::echfcs résultant en utilisant les oligonucléotides
p6apraMCS_for (5'-GCGAGCACCGGAGGCAGG-3') et
p6apraMCS_rev(5'-CATTCATCCGGGGTCAGCAC-3')
(b9) cloner le produit de l'ACP à extrémités franches dans le plasmide suicide p6suigusA::Δvdh avec HincII, pour atteindre une intégration au niveau du locus du Δvdh;
(b10) transférer le plasmide p6suiΔvdh::permE*::echfcs résultant vers le Δvdh d'Amycolatopsis sp. ATCC 39116 ;
(b11) sélectionner des transformants d'Amycolatopsis sp. ATCC 39116 en fonction du génotype correct et les cultiver pour obtenir un deuxième événement recombinant.


### SEQUENCE LISTING

<110> Symrise AG
<120> VANILLIN EX AMYCOLATOPSIS
<130> C 3286 EP
<140> EP14186798.0
   <141> 2014-09-29
<150> EP14186796.0
   <151> 2014-09-29
<160> 6
<170> BiSSAP 1.3
<210> 1
   <211> 3575
   <212> DNA
   <213> Amycolatopsis
<220>
   <223> >p6sui
<400> 1
   cagctactgg gctatctgga caagggaaaa cgcaagcgca aagagaaagc aggtagcttg      60
   cagtgggctt acatggcgat agctagactg ggcggtttta tggacagcaa gcgaaccgga     120
   attgccagct ggggcgccct ctggtaaggt tgggaagccc tgcaaagtaa actggatggc     180
   tttcttgccg ccaaggatct gatggcgcag gggatcaaga tccccgtagc ggggatccgg     240
   ggaattcccc aatgtcaagc acttccggaa tcgggagcgc ggccgatgca aagtgccgat     300
   aaacataacg atctttgtag aaaccatcgg cgcagctatt tacccgcagg acatatccac     360
   gccctcctac atcgaagctg aaagcacgag attcttcgcc ctccgagagc tgcatcaggt     420
   cggagacgct gtcgaacttt tcgatcagaa acttctcgac agacgtcgcg gtgagttcag     480
   gctttttcat atctcattgc ccccggacga gcgtctgctc cgccattcgc cgtccgccgt      540
   gccaatcgga tcagccgtcc aaatgcggga ttttcgttag tcggaggcca aacggcattg     600
   agcgtcagca tatcatcagc gagctgaaga aagacaatcc ccgatccgct ccacgtgttg     660
   ccccagcaat cagcgcgacc ttgcccctcc aacgtcatct cgttctccgc tcatgagctc     720
   agccaatcga ctggcgagcg gcatcgcatt cttcgcatcc cgcctctggc ggatgcagga     780
   agatcaacgg atctcggccc agttgaccca gggctgtcgc cacaatgtcg cgggagcgga     840
   tcaaccgagc aaaggcatga ccgactggac cttccttctg aaggctcttc tccttgagcc     900
   acctgtccgc caaggcaaag cgctcacagc agtggtcatt ctcgagataa tcgacgcgta     960
   ccaacttgcc atcctgaaga atggtgcagt gtctcggcac cccataggga acctttgcca    1020
   tcaactcggc aagatgcagc gtcgtgttgg catcgtgtcc cacgccgagg agaagtacct    1080
   gcccatcgag ttcatggaca cgggcgaccg ggcttgcagg cgagtgaggt ggcaggggca 1140
   atggatcaga gatgatctgc tctgcctgtg gccccgctgc cgcaaaggca aatggatggg 1200
   cgctgcgctt tacatttggc aggcgccaga atgtgtcaga gacaactcca aggtccggtg 1260
   taacgggcga cgtggcagga tcgaacggct cgtcgtccag acctgaccac gagggcatga 1320
   cgagcgtccc tcccggaccc agcgcagcac gcagggcctc gatcagtcca agtggcccat 1380
   cttcgagggg ccggacgcta cggaaggagc tgtggaccag cagcacaccg ccgggggtaa 1440
   ccccaaggtt gagaagctga ccgatgagct cggcttttcg ccattcgtat tgcacgacat 1500
   tgcactccac cgctgatgac atcagtcgat catagcacga tcaacggcac tgttgcaaat 1560
   agtcggtggt gataaactta tcatcccctt ttgctgatgg agctgcacat gaaccgagcg 1620
   ggctcgatcc cctcgcgagt tggttcagct gctgcctgag gctggacgac ctcgcggagt 1680
   tctaccggca gtgcaaatcc gtcggcatcc aggaaaccag cagcggctat ccgcgcatcc 1740
   atgcccccga actgcaggag tggggaggca cgatggccgc tttggtcgat actggcttaa 1800
   ctatgcggca tcagagcaga ttgtactgag agtgcaccat atgcggtgtg aaataccgca 1860
   cagatgcgta aggagaaaat accgcatcag gcgctcttcc gcttcctcgc tcactgactc 1920
   gctgcgctcg gtcgttcggc tgcggcgagc ggtatcagct cactcaaagg cggtaatacg 1980
   gttatccaca gaatcagggg ataacgcagg aaagaacatg tgagcaaaag gccagcaaaa 2040
   ggccaggaac cgtaaaaagg ccgcgttgct ggcgtttttc cataggctcc gcccccctga 2100
   cgagcatcac aaaaatcgac gctcaagtca gaggtggcga aacccgacag gactataaag 2160
   ataccaggcg tttccccctg gaagctccct cgtgcgctct cctgttccga ccctgccgct 2220
   taccggatac ctgtccgcct ttctcccttc gggaagcgtg gcgctttctc atagctcacg 2280
   ctgtaggtat ctcagttcgg tgtaggtcgt tcgctccaag ctgggctgtg tgcacgaacc 2340
   ccccgttcag cccgaccgct gcgccttatc cggtaactat cgtcttgagt ccaacccggt 2400
   aagacacgac ttatcgccac tggcagcagc cactggtaac aggattagca gagcgaggta 2460
   tgtaggcggt gctacagagt tcttgaagtg gtggcctaac tacggctaca ctagaaggac 2520
   agtatttggt atctgcgctc tgctgaagcc agttaccttc ggaaaaagag ttggtagctc 2580
   ttgatccggc aaacaaacca ccgctggtag cggtggtttt tttgtttgca agcagcagat 2640
   tacgcgcaga aaaaaaggat ctcaagaaga tcctttgatc ttttctacgg ggtctgacgc 2700
   tcagtggaac gaaaactcac gttaagggat tttggtcatg agattatcaa aaaggatctt 2760
   cacctagcca cccgatgagc cgccgccgcg ccgtccagcg aggagcccgc cgggccgagc 2820
   ccgtcgaccc gcacgcgtcc atcgagggcc agctggccct gttcggcgac gacccgcagc 2880
   ccgagcccgt caaggccgag ctggccgccg tcgacccggc ggaggcgtcc cgatgaaccc 2940
   gcgggcgagc ggcaccggat cgaggttggc ccgtacacgg cgatgctggt cacgggggcg    3000
   atccagtggg cgatccggcg cggtgagctg gccgagcacg ggccggtgat gttcgagcgg 3060
   ctgctggacc agctccggct gctgttcgcc gacgacccga ggggcgcgga gctgatctat 3120
   atctatgatc tcgcagtctc cggcgagcac cggaggcagg gcattgccac cgtccaacgc 3180
   gcttggtgct tatgtgatct acgtgcaagc agattacggt gacgatcccg cagtggctct 3240
   ctatacaaag ttgggcatac gggaagaagt gatgcacttt gatatcgtcc attccgacag 3300
   catcgccagt cactatggcg tgctgctagc gctatatgcg ttgatgcaat ttctatgcgc 3360
   acccgttctc ggagcactgt ccgaccgctt tggccgccgc ccagtcctgc tcgcttcgct 3420
   acttggagcc actatcgact acgcgatcat ggcgaccaca cccgtcctgt ggatctagaa 3480
   ttcagatcct ttaaagcttc acgctgccgc aagcactcag ggcgcaaggg ctgctaaagg 3540
   aatccgcaga aacggtgctg accccggatg aatgt 3575
<210> 2
   <211> 5398
   <212> DNA
   <213> Amycolatopsis
<220>
   <223> >p6suigusA
<400> 2
   cagctactgg gctatctgga caagggaaaa cgcaagcgca aagagaaagc aggtagcttg      60
   cagtgggctt acatggcgat agctagactg ggcggtttta tggacagcaa gcgaaccgga     120
   attgccagct ggggcgccct ctggtaaggt tgggaagccc tgcaaagtaa actggatggc     180
   tttcttgccg ccaaggatct gatggcgcag gggatcaaga tccccgtagc ggggatccgg     240
   ggaattcccc aatgtcaagc acttccggaa tcgggagcgc ggccgatgca aagtgccgat     300
   aaacataacg atctttgtag aaaccatcgg cgcagctatt tacccgcagg acatatccac     360
   gccctcctac atcgaagctg aaagcacgag attcttcgcc ctccgagagc tgcatcaggt     420
   cggagacgct gtcgaacttt tcgatcagaa acttctcgac agacgtcgcg gtgagttcag     480
   gctttttcat atctcattgc ccccggacga gcgtctgctc cgccattcgc cgtccgccgt     540
   gccaatcgga tcagccgtcc aaatgcggga ttttcgttag tcggaggcca aacggcattg     600
   agcgtcagca tatcatcagc gagctgaaga aagacaatcc ccgatccgct ccacgtgttg     660
   ccccagcaat cagcgcgacc ttgcccctcc aacgtcatct cgttctccgc tcatgagctc     720
   agccaatcga ctggcgagcg gcatcgcatt cttcgcatcc cgcctctggc ggatgcagga     780
   agatcaacgg atctcggccc agttgaccca gggctgtcgc cacaatgtcg cgggagcgga     840
   tcaaccgagc aaaggcatga ccgactggac cttccttctg aaggctcttc tccttgagcc     900
   acctgtccgc caaggcaaag cgctcacagc agtggtcatt ctcgagataa tcgacgcgta     960
   ccaacttgcc atcctgaaga atggtgcagt gtctcggcac cccataggga acctttgcca 1020
   tcaactcggc aagatgcagc gtcgtgttgg catcgtgtcc cacgccgagg agaagtacct 1080
   gcccatcgag ttcatggaca cgggcgaccg ggcttgcagg cgagtgaggt ggcaggggca 1140
   atggatcaga gatgatctgc tctgcctgtg gccccgctgc cgcaaaggca aatggatggg 1200
   cgctgcgctt tacatttggc aggcgccaga atgtgtcaga gacaactcca aggtccggtg 1260
   taacgggcga cgtggcagga tcgaacggct cgtcgtccag acctgaccac gagggcatga 1320
   cgagcgtccc tcccggaccc agcgcagcac gcagggcctc gatcagtcca agtggcccat 1380
   cttcgagggg ccggacgcta cggaaggagc tgtggaccag cagcacaccg ccgggggtaa 1440
   ccccaaggtt gagaagctga ccgatgagct cggcttttcg ccattcgtat tgcacgacat 1500
   tgcactccac cgctgatgac atcagtcgat catagcacga tcaacggcac tgttgcaaat 1560
   agtcggtggt gataaactta tcatcccctt ttgctgatgg agctgcacat gaaccgagcg 1620
   ggctcgatcc cctcgcgagt tggttcagct gctgcctgag gctggacgac ctcgcggagt 1680
   tctaccggca gtgcaaatcc gtcggcatcc aggaaaccag cagcggctat ccgcgcatcc 1740
   atgcccccga actgcaggag tggggaggca cgatggccgc tttggtcgat actggcttaa 1800
   ctatgcggca tcagagcaga ttgtactgag agtgcaccat atgcggtgtg aaataccgca 1860
   cagatgcgta aggagaaaat accgcatcag gcgctcttcc gcttcctcgc tcactgactc 1920
   gctgcgctcg gtcgttcggc tgcggcgagc ggtatcagct cactcaaagg cggtaatacg 1980
   gttatccaca gaatcagggg ataacgcagg aaagaacatg tgagcaaaag gccagcaaaa 2040
   ggccaggaac cgtaaaaagg ccgcgttgct ggcgtttttc cataggctcc gcccccctga 2100
   cgagcatcac aaaaatcgac gctcaagtca gaggtggcga aacccgacag gactataaag 2160
   ataccaggcg tttccccctg gaagctccct cgtgcgctct cctgttccga ccctgccgct 2220
   taccggatac ctgtccgcct ttctcccttc gggaagcgtg gcgctttctc atagctcacg 2280
   ctgtaggtat ctcagttcgg tgtaggtcgt tcgctccaag ctgggctgtg tgcacgaacc 2340
   ccccgttcag cccgaccgct gcgccttatc cggtaactat cgtcttgagt ccaacccggt 2400
   aagacacgac ttatcgccac tggcagcagc cactggtaac aggattagca gagcgaggta 2460
   tgtaggcggt gctacagagt tcttgaagtg gtggcctaac tacggctaca ctagaaggac 2520
   agtatttggt atctgcgctc tgctgaagcc agttaccttc ggaaaaagag ttggtagctc 2580
   ttgatccggc aaacaaacca ccgctggtag cggtggtttt tttgtttgca agcagcagat 2640
   tacgcgcaga aaaaaaggat ctcaagaaga tcctttgatc ttttctacgg ggtctgacgc 2700
   tcagtggaac gaaaactcac gttaagggat tttggtcatg agattatcaa aaaggatctt 2760
   cacctagcca cccgatgagc cgccgccgcg ccgtccagcg aggagcccgc cgggccgagc 2820
   ccgtcgaccc gcacgcgtcc atcgagggcc agctggccct gttcggcgac gacccgcagc 2880
   ccgagcccgt caaggccgag ctggccgccg tcgacccggc ggaggcgtcc cgatgaaccc 2940
   gcgggcgagc ggcaccggat cgaggttggc ccgtacacgg cgatgctggt cacgggggcg 3000
   atccagtggg cgatccggcg cggtgagctg gccgagcacg ggccggtgat gttcgagcgg 3060
   ctgctggacc agctccggct gctgttcgcc gacgacccga ggggcgcgga gctgatctat 3120
   atctatgatc tcgcagtctc cggcgagcac cggaggcagg gcattgccac cgtccaacgc 3180
   gcttggtgct tatgtgatct acgtgcaagc agattacggt gacgatcccg cagtggctct 3240
   ctatacaaag ttgggcatac gggaagaagt gatgcacttt gatatcgtcc attccgacag 3300
   catcgccagt cactatggcg tgctgctaga ggatccattg gtaccattac cctgttatcc 3360
   ctagaacgtc cgggcttgca cctcacgtca cgtgaggagg cagcgtggac ggcgtggtac 3420
   caagcttatt ggcactagtc gagcaacgga ggtacggaca tgctgcggcc cgtcgaaacc 3480
   ccgacccgcg agatcaagaa gctggacggc ctgtgggcgt tcagcctcga ccgggagaac 3540
   tgcggtatcg accagcggtg gtgggagagc gccctccagg agtcgcgcgc catcgccgtc 3600
   cccggcagct tcaacgacca gttcgcggac gcggacatcc gcaactacgc ggggaacgtc 3660
   tggtatcagc gggaggtgtt catccccaag ggctgggccg gtcagcgcat cgtgctgcgc 3720
   ttcgacgccg tgacccacta cggcaaggtc tgggtcaaca accaggaagt gatggagcac 3780
   cagggcggct acacgccctt cgaggcggac gtgaccccgt acgtcatcgc cggcaagtcg 3840
   gtccgcatca cggtctgcgt caacaacgag ctcaactggc agaccatccc gcccggcatg 3900
   gtgatcacgg acgagaacgg caagaagaag cagagctact tccacgactt cttcaactac 3960
   gccggcatcc accgctcggt catgctgtac acgacgccga acacctgggt cgacgacatc 4020
   acggtggtca cccacgtggc ccaggactgc aaccacgcgt ccgtggactg gcaggtcgtc 4080
   gccaacggcg acgtgagcgt ggagctccgc gacgccgagc agcaggtcgt ggcgaccggg 4140
   cagggcacct cggggaccct ccaggtcgtg aacccccacc tctggcagcc gggtgagggc 4200
   tacctgtacg agctgtgcgt gacggcgaag agccagaccg agtgcgacat ctaccccctg 4260
   cgcgtcggca tccggtccgt ggcggtcaag ggcgagcagt tcctgatcaa ccacaagccg 4320
   ttctacttca cgggcttcgg tcggcacgag gacgccgacc tccgcggcaa gggcttcgac 4380
   aacgtcctga tggtccacga ccacgccctg atggactgga tcggcgccaa ctcgtaccgg 4440
   acctcgcact acccgtacgc ggaagagatg ctggactggg cggacgagca cgggatcgtc 4500
   gtcatcgacg aaaccgccgc ggtgggcttc aacctgtccc tcggcatcgg cttcgaggcc 4560
   ggcaacaagc cgaaagagct ctactccgaa gaggccgtca acggcgaaac ccagcaggcc 4620
   cacctccagg cgatcaaaga gctcatcgcc cgcgacaaga accacccgtc cgtcgtgatg 4680
   tggtcgatcg ccaacgagcc ggacacccgg ccgcagggtg cccgggagta cttcgccccg 4740
   ctggccgagg ccacccggaa gctcgacccc acccggccca tcacctgcgt gaacgtgatg      4800
   ttctgcgacg cccacacgga caccatctcc gacctcttcg acgtcctgtg cctgaaccgc      4860
   tactacggct ggtacgtgca gtccggggac ctggaaacgg ccgagaaggt cctggagaaa      4920
   gagctgctgg cgtggcagga gaagctgcac cagccgatca tcatcaccga gtacggcgtg      4980
   gacacgctgg ccggtctgca ctccatgtac accgacatgt ggtccgaaga gtaccagtgc      5040
   gcgtggctgg acatgtacca ccgcgtgttc gaccgcgtca gcgcggtcgt cggcgagcag      5100
   gtgtggaact tcgccgactt cgccacctcc cagggcatcc tccgcgtcgg gggcaacaag      5160
   aagggcatct tcacccggga ccgcaagccc aagtcggcgg cgttcctgct ccagaagcgg      5220
   tggaccggga tgaacttcgg ggagaagccg cagcagggcg ggaagcagtg ataagcatta      5280
   ccctgttatc cctaggatcc gcggccgcgc gcgataaagc ttcacgctgc cgcaagcact      5340
   cagggcgcaa gggctgctaa aggaatccgc agaaacggtg ctgaccccgg atgaatgt        5398
<210> 3
   <211> 7103
   <212> DNA
   <213> Amycolatopsis
<220>
   <223> p6suigusA::deltavdh
<400> 3
   cagctactgg gctatctgga caagggaaaa cgcaagcgca aagagaaagc aggtagcttg      60
   cagtgggctt acatggcgat agctagactg ggcggtttta tggacagcaa gcgaaccgga     120
   attgccagct ggggcgccct ctggtaaggt tgggaagccc tgcaaagtaa actggatggc     180
   tttcttgccg ccaaggatct gatggcgcag gggatcaaga tccccgtagc ggggatccgg     240
   ggaattcccc aatgtcaagc acttccggaa tcgggagcgc ggccgatgca aagtgccgat     300
   aaacataacg atctttgtag aaaccatcgg cgcagctatt tacccgcagg acatatccac     360
   gccctcctac atcgaagctg aaagcacgag attcttcgcc ctccgagagc tgcatcaggt     420
   cggagacgct gtcgaacttt tcgatcagaa acttctcgac agacgtcgcg gtgagttcag     480
   gctttttcat atctcattgc ccccggacga gcgtctgctc cgccattcgc cgtccgccgt      540
   gccaatcgga tcagccgtcc aaatgcggga ttttcgttag tcggaggcca aacggcattg     600
   agcgtcagca tatcatcagc gagctgaaga aagacaatcc ccgatccgct ccacgtgttg     660
   ccccagcaat cagcgcgacc ttgcccctcc aacgtcatct cgttctccgc tcatgagctc     720
   agccaatcga ctggcgagcg gcatcgcatt cttcgcatcc cgcctctggc ggatgcagga     780
   agatcaacgg atctcggccc agttgaccca gggctgtcgc cacaatgtcg cgggagcgga     840
   tcaaccgagc aaaggcatga ccgactggac cttccttctg aaggctcttc tccttgagcc     900
   acctgtccgc caaggcaaag cgctcacagc agtggtcatt ctcgagataa tcgacgcgta     960
   ccaacttgcc atcctgaaga atggtgcagt gtctcggcac cccataggga acctttgcca 1020
   tcaactcggc aagatgcagc gtcgtgttgg catcgtgtcc cacgccgagg agaagtacct 1080
   gcccatcgag ttcatggaca cgggcgaccg ggcttgcagg cgagtgaggt ggcaggggca 1140
   atggatcaga gatgatctgc tctgcctgtg gccccgctgc cgcaaaggca aatggatggg 1200
   cgctgcgctt tacatttggc aggcgccaga atgtgtcaga gacaactcca aggtccggtg 1260
   taacgggcga cgtggcagga tcgaacggct cgtcgtccag acctgaccac gagggcatga 1320
   cgagcgtccc tcccggaccc agcgcagcac gcagggcctc gatcagtcca agtggcccat 1380
   cttcgagggg ccggacgcta cggaaggagc tgtggaccag cagcacaccg ccgggggtaa 1440
   ccccaaggtt gagaagctga ccgatgagct cggcttttcg ccattcgtat tgcacgacat 1500
   tgcactccac cgctgatgac atcagtcgat catagcacga tcaacggcac tgttgcaaat 1560
   agtcggtggt gataaactta tcatcccctt ttgctgatgg agctgcacat gaaccgagcg 1620
   ggctcgatcc cctcgcgagt tggttcagct gctgcctgag gctggacgac ctcgcggagt 1680
   tctaccggca gtgcaaatcc gtcggcatcc aggaaaccag cagcggctat ccgcgcatcc 1740
   atgcccccga actgcaggag tggggaggca cgatggccgc tttggtcgat actggcttaa 1800
   ctatgcggca tcagagcaga ttgtactgag agtgcaccat atgcggtgtg aaataccgca 1860
   cagatgcgta aggagaaaat accgcatcag gcgctcttcc gcttcctcgc tcactgactc 1920
   gctgcgctcg gtcgttcggc tgcggcgagc ggtatcagct cactcaaagg cggtaatacg 1980
   gttatccaca gaatcagggg ataacgcagg aaagaacatg tgagcaaaag gccagcaaaa 2040
   ggccaggaac cgtaaaaagg ccgcgttgct ggcgtttttc cataggctcc gcccccctga 2100
   cgagcatcac aaaaatcgac gctcaagtca gaggtggcga aacccgacag gactataaag 2160
   ataccaggcg tttccccctg gaagctccct cgtgcgctct cctgttccga ccctgccgct 2220
   taccggatac ctgtccgcct ttctcccttc gggaagcgtg gcgctttctc atagctcacg 2280
   ctgtaggtat ctcagttcgg tgtaggtcgt tcgctccaag ctgggctgtg tgcacgaacc 2340
   ccccgttcag cccgaccgct gcgccttatc cggtaactat cgtcttgagt ccaacccggt 2400
   aagacacgac ttatcgccac tggcagcagc cactggtaac aggattagca gagcgaggta 2460
   tgtaggcggt gctacagagt tcttgaagtg gtggcctaac tacggctaca ctagaaggac 2520
   agtatttggt atctgcgctc tgctgaagcc agttaccttc ggaaaaagag ttggtagctc 2580
   ttgatccggc aaacaaacca ccgctggtag cggtggtttt tttgtttgca agcagcagat 2640
   tacgcgcaga aaaaaaggat ctcaagaaga tcctttgatc ttttctacgg ggtctgacgc 2700
   tcagtggaac gaaaactcac gttaagggat tttggtcatg agattatcaa aaaggatctt 2760
   cacctagcca cccgatgagc cgccgccgcg ccgtccagcg aggagcccgc cgggccgagc 2820
   ccgtcgaccc gcacgcgtcc atcgagggcc agctggccct gttcggcgac gacccgcagc 2880
   ccgagcccgt caaggccgag ctggccgccg tcgacccggc ggaggcgtcc cgatgaaccc 2940
   gcgggcgagc ggcaccggat cgaggttggc ccgtacacgg cgatgctggt cacgggggcg 3000
   atccagtggg cgatccggcg cggtgagctg gccgagcacg ggccggtgat gttcgagcgg 3060
   ctgctggacc agctccggct gctgttcgcc gacgacccga ggggcgcgga gctgatctat 3120
   atctatgatc tcgcagtctc cggcgagcac cggaggcagg gcattgccac cgtccaacgc 3180
   gcttggtgct tatgtgatct acgtgcaagc agattacggt gacgatcccg cagtggctct 3240
   ctatacaaag ttgggcatac gggaagaagt gatgcacttt gatgatcttc cggatggctc 3300
   gagtttttca gcaagattcg tgtgggtgtt cgggtacggg ttcgtggcgc ggatgcggcc 3360
   gggcgaacgc acgccgtggt cgtacttcgc ggtgatctcg tgggccggga tgcgcggcgt 3420
   ggtcacgctc gccgccgcgt tcgtgctccc ggacgacacg ccgcagcgcg cggtgctggt 3480
   gctggcggcg ttcgtcgtcg tggccggaac gctgctgctg cacggcatga ccctgcccgg 3540
   cctggtgcgc cgcctgcgcc tgccgccgcc caacccggcg gaggacgcgc tgcaggaagc 3600
   cgcgctgctg cacgacatga cgtccgcggg cctggcccgg ctcgaggaga tccgccgccc 3660
   ggccgacccg cccgaggtca tcgaccggtt gcgggacaag cagtcctacc ggtcggactc 3720
   ggcgtgggag aacctcggca ggctcagcgc gctggaggag acccccagcg acgcgtaccg 3780
   tcggctgcgc gccgagatgc tcgcggccga gcgggacgtc ctgctcaccg cgcgggaccg 3840
   gggcaccgcc gacgacgagg tgctccggcg ggtgatggac ggactggaca tcgaggagtc 3900
   gctgctggag cgacccgagg aggagccggg catggaacgt gagctggaga cccccgaggc 3960
   cacggcgggc acctgcgagc acctggagaa ggcacgcgtg ccggagaccg acgccgaggg 4020
   ctgcgtcgac tgcctgcgcg agggctcgtc gtgggtgcac ctgcggctgt gcctggagtg 4080
   cgggcacgtc ggctgctgcg actcgtcgcc gcgcaagcac gccacgcagc acttccacga 4140
   cacgcgtcac ccggtgatcc gcagctacga gcccggcgag aactggcgct ggtgcttcgt 4200
   ggacgggatc gtcggctgag cgggggcgcc gtcggcgccc ccgccgcgcg tgtcactcct 4260
   gatgcgcgag gttttccgcc atcctcctgg gctgttcggg ccgcgccagt gggtctttca 4320
   ctgagtggaa ggccggaaaa ctgtcggtgg tggctgcgat gatcgcgctc gtggacgact 4380
   cgaagatccg gcggacgctc gcctcgcacg gcccggtgcg gcggcggcca cgggcggtgc 4440
   tcgaacggat gctggcgctg gcgggggagg acctgtcgcc cgcggagccg gtggcggcac 4500
   tggaggcgcg gatcgcgtcg ctgctgggca agccggcggc gttgttcttc ccgagcggga 4560
   cgatggcgca gcaggcggcg ctgcgggtgc acgcggaccg gtccgggcgg cggacgttcg 4620
   ccgcgcaccc gtacacgcat ctggacaact gggaggacaa ggggtacgcg gcggtgcacg 4680
   ggctgcggtt ccggccggtg gggaccgcaa acgagctgat gacgccggcg gacctggagg 4740
   cggtggggga gccgctcgcg gccgtggtgt gggagctgcc gcagcgggac ctcggcgggt 4800
   tgctaccgtc gtgggacgag ctggtcgcgc agaccgggct cgcgcgctcc cgcggcgcgg 4860
   cggcgcacct ggacggcgct cggctgtggg agtcgcaacc gttctacggc aaggatttcg 4920
   ccgagatcgc cggcctgttc gacagcgtgt acgtgtcgct ctacaaagga ctgcaaatct 4980
   ttctagaaat cgtccattcc gacagcatcg ccagtcacta tggcgtgctg ctagaggatc 5040
   cattggtacc attaccctgt tatccctaga acgtccgggc ttgcacctca cgtcacgtga 5100
   ggaggcagcg tggacggcgt ggtaccaagc ttattggcac tagtcgagca acggaggtac 5160
   ggacatgctg cggcccgtcg aaaccccgac ccgcgagatc aagaagctgg acggcctgtg 5220
   ggcgttcagc ctcgaccggg agaactgcgg tatcgaccag cggtggtggg agagcgccct 5280
   ccaggagtcg cgcgccatcg ccgtccccgg cagcttcaac gaccagttcg cggacgcgga 5340
   catccgcaac tacgcgggga acgtctggta tcagcgggag gtgttcatcc ccaagggctg 5400
   ggccggtcag cgcatcgtgc tgcgcttcga cgccgtgacc cactacggca aggtctgggt 5460
   caacaaccag gaagtgatgg agcaccaggg cggctacacg cccttcgagg cggacgtgac 5520
   cccgtacgtc atcgccggca agtcggtccg catcacggtc tgcgtcaaca acgagctcaa 5580
   ctggcagacc atcccgcccg gcatggtgat cacggacgag aacggcaaga agaagcagag 5640
   ctacttccac gacttcttca actacgccgg catccaccgc tcggtcatgc tgtacacgac 5700
   gccgaacacc tgggtcgacg acatcacggt ggtcacccac gtggcccagg actgcaacca 5760
   cgcgtccgtg gactggcagg tcgtcgccaa cggcgacgtg agcgtggagc tccgcgacgc 5820
   cgagcagcag gtcgtggcga ccgggcaggg cacctcgggg accctccagg tcgtgaaccc 5880
   ccacctctgg cagccgggtg agggctacct gtacgagctg tgcgtgacgg cgaagagcca 5940
   gaccgagtgc gacatctacc ccctgcgcgt cggcatccgg tccgtggcgg tcaagggcga 6000
   gcagttcctg atcaaccaca agccgttcta cttcacgggc ttcggtcggc acgaggacgc 6060
   cgacctccgc ggcaagggct tcgacaacgt cctgatggtc cacgaccacg ccctgatgga 6120
   ctggatcggc gccaactcgt accggacctc gcactacccg tacgcggaag agatgctgga 6180
   ctgggcggac gagcacggga tcgtcgtcat cgacgaaacc gccgcggtgg gcttcaacct 6240
   gtccctcggc atcggcttcg aggccggcaa caagccgaaa gagctctact ccgaagaggc 6300
   cgtcaacggc gaaacccagc aggcccacct ccaggcgatc aaagagctca tcgcccgcga 6360
   caagaaccac ccgtccgtcg tgatgtggtc gatcgccaac gagccggaca cccggccgca 6420
   gggtgcccgg gagtacttcg ccccgctggc cgaggccacc cggaagctcg accccacccg 6480
   gcccatcacc tgcgtgaacg tgatgttctg cgacgcccac acggacacca tctccgacct 6540
   cttcgacgtc ctgtgcctga accgctacta cggctggtac gtgcagtccg gggacctgga 6600
   aacggccgag aaggtcctgg agaaagagct gctggcgtgg caggagaagc tgcaccagcc      6660
   gatcatcatc accgagtacg gcgtggacac gctggccggt ctgcactcca tgtacaccga      6720
   catgtggtcc gaagagtacc agtgcgcgtg gctggacatg taccaccgcg tgttcgaccg      6780
   cgtcagcgcg gtcgtcggcg agcaggtgtg gaacttcgcc gacttcgcca cctcccaggg      6840
   catcctccgc gtcgggggca acaagaaggg catcttcacc cgggaccgca agcccaagtc      6900
   ggcggcgttc ctgctccaga agcggtggac cgggatgaac ttcggggaga agccgcagca      6960
   gggcgggaag cagtgataag cattaccctg ttatccctag gatccgcggc cgcgcgcgat      7020
   aaagcttcac gctgccgcaa gcactcaggg cgcaagggct gctaaaggaa tccgcagaaa      7080
   cggtgctgac cccggatgaa tgt                                              7103
<210> 4
   <211> 5476
   <212> DNA
   <213> Amycolatopsis
<220>
   <223> >pSK::echfcs
<400> 4
   cacctgacgc gccctgtagc ggcgcattaa gcgcggcggg tgtggtggtt acgcgcagcg       60
   tgaccgctac acttgccagc gccctagcgc ccgctccttt cgctttcttc ccttcctttc      120
   tcgccacgtt cgccggcttt ccccgtcaag ctctaaatcg ggggctccct ttagggttcc      180
   gatttagtgc tttacggcac ctcgacccca aaaaacttga ttagggtgat ggttcacgta      240
   gtgggccatc gccctgatag acggtttttc gccctttgac gttggagtcc acgttcttta      300
   atagtggact cttgttccaa actggaacaa cactcaaccc tatctcggtc tattcttttg      360
   atttataagg gattttgccg atttcggcct attggttaaa aaatgagctg atttaacaaa      420
   aatttaacgc gaattttaac aaaatattaa cgcttacaat ttccattcgc cattcaggct      480
   gcgcaactgt tgggaagggc gatcggtgcg ggcctcttcg ctattacgcc agctggcgaa      540
   agggggatgt gctgcaaggc gattaagttg ggtaacgcca gggttttccc agtcacgacg      600
   ttgtaaaacg acggccagtg aattgtaata cgactcacta tagggcgaat tgggtaccgg      660
   gccccccctc gaggtcgacg gtatcgataa gcttgatatc gaattcctgc agcccggggg      720
   atccactagt tctagaccac agagtagccg cagcgggccg gcagggccgc atgagcgggg      780
   ttcatcggcc cctggtcagc cgaagcggcg gcggacctcg cccttgagga tcttgccgga      840
   gccggtcgtg ggcagccggt cggtgaactc gaccgacttg ggcaccttgt acccggccag      900
   ccgggtgcgc agccaggcca gcagctcgtc gccgtcgatg cggctcccgt cggcgggcac      960
   gacgaccgcc ttgcccacct cgccccagcg cgggtccggc acgccgatca cggcgcacgc     1020
   ctccacaccc gggtagccgt acagctcgtt ctccacctcg gccgggtaga tgttctcgcc     1080
   gccggagatg atcatgtcct tgagccggtc gacgacgtgg aagtacccgt cgccgtccac 1140
   ggtggccacg tcgccggagt ggaaccaccc gtcgcgcagc acctcggcgg tcgcctccgg 1200
   cctgccccag tagcccttca tcacgttggg cccgctgacc acgatctcgc ccttctcccc 1260
   cggcggcacc ggctcgccgg acgggccggc cagccgcacg tcggtgaaga acgagggcac 1320
   cccggcggag ccgatcttct ccgcgacgtg cgcgcggtcg aggaccagca cgcccggcgc 1380
   ggcctcggtc atgccgtagc cctgcacgaa cgcgagcccg cggtcgaggt agcggctggc 1440
   gaggtcggcg ggcaccggcg cgccgccgca cagcagggtc cgcaggctgg acaggtcggc 1500
   gctgcgccac cgcgggtgcg cggcgatcgc ctggtacatc gtgggcacgc cgaacacgag 1560
   cgtgacccgc tcctgttcca ccgcggacag cacggcgccg gggtcgaacg cggagtgcag 1620
   gatcaccgtg ccgcccttga gcagggtggg caggcacacc atgccgagcg cggcggcgtg 1680
   gaacagcggc gcggcgacca gtgcccgctc gtcgctcgcc aggtcggtct ccaccaggac 1740
   gttgacgcag ttccaggtga ggttgccgtg ggtgagcatc gcgcccttgg ggcgtccggt 1800
   gctgcccgag gtgtacatca gcaggcacac gtcgtcgagg ccgacgggca ggtcgggcgg 1860
   ctcgtcgtcc gcggcgtcca gctcgcccgc gggcacgcgc agcagccggc cggcggcgag 1920
   cgccgcgacc gtctccgcgt gctccggggt gtggataagg accgacgcgc cggagtcggc 1980
   cagcgcgtgg tccagttccg ggacgcccag ccggaagttc agcggcacga acaccgcgcc 2040
   ggcctgcccg cacgcgaaca gggtctccag gtaggccggg tggttcggcc cgaggtaggc 2100
   cacccggtcg ccggggcgga ccccggcggc ccgcagcccg ttggcgagcc gcgcgacgcg 2160
   gcgggacagc tcggcgtagg tcagcgccgt cccgccgtgc cggacggctg tcgcgtgcgg 2220
   gctcatcctg gcgcggcgca ccggccagga gcccagaccc tggttgcgca ctacttctcc 2280
   gggtcgaagg cgctcaggcc gggccggtag gacttctggt cgaggaactg ggtcagcccc 2340
   ttggcgcggg cgcccgcctt gtcggcgaac tgggactggt cgagcttggc gtagaggtag 2400
   tcctcggcct gctcccaggg catctcctgg gcgatcttgt acccggtctt ggccgcgtgc 2460
   aggaccacct ggttcatcga cgcgagcttc agcgccacct cgcgggtgcg ctcccgcagc 2520
   cggtcggcgg gcagcgcctc gttgaccagg cgcatctccg cgcgcggcgg gccgtcgaag 2580
   ggctcaccgg tcatgatgta gtacagcgcg tcgcgctgcg gcacggtcgc cgccagcgcc 2640
   cggctgacca cgccgcccgg cgggatgccc cagttgacct cggacagccc gaaccgcgcg 2700
   tcctcgtcgg cgaaggccag gtcgcaggcc accagcgggg tgaacgcgcc gccgaagcac 2760
   cagccgttga ccatcgcgat cgtcggcttg ctccagttcg ccagccgctt ccactgccac 2820
   tccgcgctgg cccgccgcac cttgatctgc acggcggtgc tgccggtggc gtcgacctcg 2880
   cggaagtact ccttgaggtc catgcccgcg gagaacgact cgcccgcgcc ggtcagcacc 2940
   agcactcggc agcggtcgtc gccctccagg tggtccagca cccgcaccat ctcgtcgttc 3000
   agggtggggt tcatggcgtt gcgcttgtcc ggccggttga gcatgaccca ggcgatgcct 3060
   tcgtcgaact ccaccagaac cgtctcgccc cacggctccg tccggacccg cccgttgccg 3120
   accgctgtgc tcatgcatcg ctccctatat cagtagttct gatataggga tagcaagagc 3180
   gggccgacgg cgtcaaggtc agatggccgg cgcgccgttc aggacggcgg cgaccagccc 3240
   gccgagcgcg gccagcagtt gtcacctcct tagagctcca gcttttgttc cctttagtga 3300
   gggttaattt cgagcttggc gtaatcatgg tcatagctgt ttcctgtgtg aaattgttat 3360
   ccgctcacaa ttccacacaa catacgagcc ggaagcataa agtgtaaagc ctggggtgcc 3420
   taatgagtga gctaactcac attaattgcg ttgcgctcac tgcccgcttt ccagtcggga 3480
   aacctgtcgt gccagctgca ttaatgaatc ggccaacgcg cggggagagg cggtttgcgt 3540
   attgggcgct cttccgcttc ctcgctcact gactcgctgc gctcggtcgt tcggctgcgg 3600
   cgagcggtat cagctcactc aaaggcggta atacggttat ccacagaatc aggggataac 3660
   gcaggaaaga acatgtgagc aaaaggccag caaaaggcca ggaaccgtaa aaaggccgcg 3720
   ttgctggcgt ttttccatag gctccgcccc cctgacgagc atcacaaaaa tcgacgctca 3780
   agtcagaggt ggcgaaaccc gacaggacta taaagatacc aggcgtttcc ccctggaagc 3840
   tccctcgtgc gctctcctgt tccgaccctg ccgcttaccg gatacctgtc cgcctttctc 3900
   ccttcgggaa gcgtggcgct ttctcatagc tcacgctgta ggtatctcag ttcggtgtag 3960
   gtcgttcgct ccaagctggg ctgtgtgcac gaaccccccg ttcagcccga ccgctgcgcc 4020
   ttatccggta actatcgtct tgagtccaac ccggtaagac acgacttatc gccactggca 4080
   gcagccactg gtaacaggat tagcagagcg aggtatgtag gcggtgctac agagttcttg 4140
   aagtggtggc ctaactacgg ctacactaga aggacagtat ttggtatctg cgctctgctg 4200
   aagccagtta ccttcggaaa aagagttggt agctcttgat ccggcaaaca aaccaccgct 4260
   ggtagcggtg gtttttttgt ttgcaagcag cagattacgc gcagaaaaaa aggatctcaa 4320
   gaagatcctt tgatcttttc tacggggtct gacgctcagt ggaacgaaaa ctcacgttaa 4380
   gggattttgg tcatgagatt atcaaaaagg atcttcacct agatcctttt aaattaaaaa 4440
   tgaagtttta aatcaatcta aagtatatat gagtaaactt ggtctgacag ttaccaatgc 4500
   ttaatcagtg aggcacctat ctcagcgatc tgtctatttc gttcatccat agttgcctga 4560
   ctccccgtcg tgtagataac tacgatacgg gagggcttac catctggccc cagtgctgca 4620
   atgataccgc gagacccacg ctcaccggct ccagatttat cagcaataaa ccagccagcc 4680
   ggaagggccg agcgcagaag tggtcctgca actttatccg cctccatcca gtctattaat 4740
   tgttgccggg aagctagagt aagtagttcg ccagttaata gtttgcgcaa cgttgttgcc 4800
   attgctacag gcatcgtggt gtcacgctcg tcgtttggta tggcttcatt cagctccggt 4860
   tcccaacgat caaggcgagt tacatgatcc cccatgttgt gcaaaaaagc ggttagctcc     4920
   ttcggtcctc cgatcgttgt cagaagtaag ttggccgcag tgttatcact catggttatg     4980
   gcagcactgc ataattctct tactgtcatg ccatccgtaa gatgcttttc tgtgactggt     5040
   gagtactcaa ccaagtcatt ctgagaatag tgtatgcggc gaccgagttg ctcttgcccg     5100
   gcgtcaatac gggataatac cgcgccacat agcagaactt taaaagtgct catcattgga     5160
   aaacgttctt cggggcgaaa actctcaagg atcttaccgc tgttgagatc cagttcgatg     5220
   taacccactc gtgcacccaa ctgatcttca gcatctttta ctttcaccag cgtttctggg     5280
   tgagcaaaaa caggaaggca aaatgccgca aaaaagggaa taagggcgac acggaaatgt     5340
   tgaatactca tactcttcct ttttcaatat tattgaagca tttatcaggg ttattgtctc     5400
   atgagcggat acatatttga atgtatttag aaaaataaac aaataggggt tccgcgcaca     5460
   tttccccgaa aagtgc                                                     5476
<210> 5
   <211> 8913
   <212> DNA
   <213> Amycolatopsis
<220>
   <223> >p6permE::echfcs
<400> 5
   cagctactgg gctatctgga caagggaaaa cgcaagcgca aagagaaagc aggtagcttg      60
   cagtgggctt acatggcgat agctagactg ggcggtttta tggacagcaa gcgaaccgga     120
   attgccagct ggggcgccct ctggtaaggt tgggaagccc tgcaaagtaa actggatggc     180
   tttcttgccg ccaaggatct gatggcgcag gggatcaaga tccccgtagc ggggatccgg     240
   ggaattcccc aatgtcaagc acttccggaa tcgggagcgc ggccgatgca aagtgccgat     300
   aaacataacg atctttgtag aaaccatcgg cgcagctatt tacccgcagg acatatccac     360
   gccctcctac atcgaagctg aaagcacgag attcttcgcc ctccgagagc tgcatcaggt     420
   cggagacgct gtcgaacttt tcgatcagaa acttctcgac agacgtcgcg gtgagttcag     480
   gctttttcat atctcattgc ccccggacga gcgtctgctc cgccattcgc cgtccgccgt     540
   gccaatcgga tcagccgtcc aaatgcggga ttttcgttag tcggaggcca aacggcattg     600
   agcgtcagca tatcatcagc gagctgaaga aagacaatcc ccgatccgct ccacgtgttg     660
   ccccagcaat cagcgcgacc ttgcccctcc aacgtcatct cgttctccgc tcatgagctc     720
   agccaatcga ctggcgagcg gcatcgcatt cttcgcatcc cgcctctggc ggatgcagga     780
   agatcaacgg atctcggccc agttgaccca gggctgtcgc cacaatgtcg cgggagcgga     840
   tcaaccgagc aaaggcatga ccgactggac cttccttctg aaggctcttc tccttgagcc     900
   acctgtccgc caaggcaaag cgctcacagc agtggtcatt ctcgagataa tcgacgcgta      960
   ccaacttgcc atcctgaaga atggtgcagt gtctcggcac cccataggga acctttgcca     1020
   tcaactcggc aagatgcagc gtcgtgttgg catcgtgtcc cacgccgagg agaagtacct     1080
   gcccatcgag ttcatggaca cgggcgaccg ggcttgcagg cgagtgaggt ggcaggggca     1140
   atggatcaga gatgatctgc tctgcctgtg gccccgctgc cgcaaaggca aatggatggg     1200
   cgctgcgctt tacatttggc aggcgccaga atgtgtcaga gacaactcca aggtccggtg     1260
   taacgggcga cgtggcagga tcgaacggct cgtcgtccag acctgaccac gagggcatga     1320
   cgagcgtccc tcccggaccc agcgcagcac gcagggcctc gatcagtcca agtggcccat     1380
   cttcgagggg ccggacgcta cggaaggagc tgtggaccag cagcacaccg ccgggggtaa     1440
   ccccaaggtt gagaagctga ccgatgagct cggcttttcg ccattcgtat tgcacgacat     1500
   tgcactccac cgctgatgac atcagtcgat catagcacga tcaacggcac tgttgcaaat     1560
   agtcggtggt gataaactta tcatcccctt ttgctgatgg agctgcacat gaaccgagcg     1620
   ggctcgatcc cctcgcgagt tggttcagct gctgcctgag gctggacgac ctcgcggagt     1680
   tctaccggca gtgcaaatcc gtcggcatcc aggaaaccag cagcggctat ccgcgcatcc     1740
   atgcccccga actgcaggag tggggaggca cgatggccgc tttggtcgat actggcttaa     1800
   ctatgcggca tcagagcaga ttgtactgag agtgcaccat atgcggtgtg aaataccgca     1860
   cagatgcgta aggagaaaat accgcatcag gcgctcttcc gcttcctcgc tcactgactc     1920
   gctgcgctcg gtcgttcggc tgcggcgagc ggtatcagct cactcaaagg cggtaatacg     1980
   gttatccaca gaatcagggg ataacgcagg aaagaacatg tgagcaaaag gccagcaaaa     2040
   ggccaggaac cgtaaaaagg ccgcgttgct ggcgtttttc cataggctcc gcccccctga     2100
   cgagcatcac aaaaatcgac gctcaagtca gaggtggcga aacccgacag gactataaag     2160
   ataccaggcg tttccccctg gaagctccct cgtgcgctct cctgttccga ccctgccgct     2220
   taccggatac ctgtccgcct ttctcccttc gggaagcgtg gcgctttctc atagctcacg     2280
   ctgtaggtat ctcagttcgg tgtaggtcgt tcgctccaag ctgggctgtg tgcacgaacc     2340
   ccccgttcag cccgaccgct gcgccttatc cggtaactat cgtcttgagt ccaacccggt     2400
   aagacacgac ttatcgccac tggcagcagc cactggtaac aggattagca gagcgaggta     2460
   tgtaggcggt gctacagagt tcttgaagtg gtggcctaac tacggctaca ctagaaggac     2520
   agtatttggt atctgcgctc tgctgaagcc agttaccttc ggaaaaagag ttggtagctc     2580
   ttgatccggc aaacaaacca ccgctggtag cggtggtttt tttgtttgca agcagcagat     2640
   tacgcgcaga aaaaaaggat ctcaagaaga tcctttgatc ttttctacgg ggtctgacgc     2700
   tcagtggaac gaaaactcac gttaagggat tttggtcatg agattatcaa aaaggatctt     2760
   cacctagcca cccgatgagc cgccgccgcg ccgtccagcg aggagcccgc cgggccgagc     2820
   ccgtcgaccc gcacgcgtcc atcgagggcc agctggccct gttcggcgac gacccgcagc 2880
   ccgagcccgt caaggccgag ctggccgccg tcgacccggc ggaggcgtcc cgatgaaccc 2940
   gcggaacaac gccgacgacg ccagcgacgc cgccgtgggc tggaccgagg cccggtggct 3000
   ggtctccaac ggcaaggcga agtagtcctg accgtcgacc aggtggtgcc cgccgggccg 3060
   ttgcccggcg ggcactgtcg cgttcggatg cacggagacc aggggaaacg tggacgtctg 3120
   acgatgatcg gccgtaggat cacggacgcg cccgacgcgc aaacggtccg cgtcggccgc 3180
   gagaggcggg ttacccccgg cccgccaccc tctcgcgtgg cattcgcccg aacgtggcaa 3240
   cttttccggc gtgatcatcg gcgtgtcgct cgttttcgcg tactcgaaac gatagaacgt 3300
   gccgtatggg acgaccgaag ggcagacccc cgcgctggga cgccgacgtt cagctgaagg 3360
   tgatcaaggc ccggatgccg gtcgagctgg ccgaggcgtt ccagaaggaa gcgcagaagc 3420
   gcggcatgaa cgtgcaggac ttcctgggcc gactggccga ggagctgacc ggcgtgccct 3480
   acaacccgca gggaggtttg ccgctgagcg cctagaacga cgaaaagcgc cccgccagga 3540
   gttaccagct cccgcgagac gcttccacgc cgctaactga acatccacgc ccaccaaggc 3600
   cactgactcg tgaagggttt cgccggccgt tggtgcgctg tatctacgct tggagccacc 3660
   ataccaaccc ggcccaggtg tggcccagta ctgcccgcgt gcgacacgcg gacggattct 3720
   ccagggtcca ccgaatcagc aatcttgacg cttaagcgtc aacctgtcag cgacttgtgc 3780
   gtcagttgcg gccgggcccc tggcgtgcca cggcccaccc cgttgcggac cgtccactgt 3840
   ggcccgtgct gggccgagtt cggcgggtcg cccgacgtcg ccgagatcgg cccgcccccg 3900
   ccggaggctc tggggtcgcg cgaccccaga gaccagcggg aatggataac gagcctggcc 3960
   ggtgagcagt gggtccagat gctccggagc gactgccgcg cccgtctgct cgacctcgca 4020
   caggagatga tgctccgcgc gaactgggac acctgggagt cgtggccagg ctgggaccgg 4080
   ctcatggagg tgtcccgctg gaagcgctcc gcgctggccg gctggctgcg ccagctgcgc 4140
   ctgctcggct ggttgtcagt gatcgagtcc ggcagcacgc cgcagttccg gccgatggtc 4200
   ctgtcccacg tcgagggcaa ccgccgcgcc gtctacgccc tgcgggtgcc ccgccgtccc 4260
   ggcgagcagg ccaccgcccc ccaaccgcca gcccgtcccg agcccgagct ggagaccgtc 4320
   ccggccgagc cgttcacccg ttctgaccag cgcggacgcg cgtctgtgga tagaacctgg 4380
   acccctacgt ggttctttga tctttcttct agagttgatc tttgggtttc ttcgcgcgcg 4440
   agagattttt tccacagctc ccagctcggc gcgcaggttg agcccaagac ggagccgctt 4500
   cgcggccgct ttgacgagga atggctggcg ttcgctcgaa cgtcgccgac cagcggcgct 4560
   cagatgctcg ccgcggccgc cgagctgcgc cgccaggagc cgatcctggc ccagttgacc 4620
   gcgcgcgccg tccgggccct gtgtcgcccg tactgggctg ctggctgggc aaacgacgat 4680
   gtgcggcacg cgctccgctg gcggccgcgg gccacgtcga gcctgcccgc gatggagcag 4740
   gcgaagatcc acgcgccgta ccggtgggcg cagtcgaggc tggccgcctg gcgcaccgac 4800
   accggcaagg ttctgcctgg tcacagccac atggagcgcg cccagcggac ccgcgagcac 4860
   gcggtggtgg cccgcttcgg caaggccgcg ctggccgcga tgcccgccgg gtcggaccgg 4920
   ctgcgcccgg ttgacgtgcg ccggttcggc cgccgccggg tcgacgccgc ggtggagacg 4980
   ctgcgccgcc gtcgggccgc cgagctggcc gacgagcgcg ccgccgtccg gtcgcaggcc 5040
   gaggccgccg accccgagca cgcccgccgg gtcgccgacg agttccgcgc cgagcgcgag 5100
   cgccgccagg ccgctggccg cgagcggctg ctggagcagg cccgcgccgc ggccgccgag 5160
   cgggccgagc aggaccagcc cgccccggcg agcacgtgga caccgcaggg accagcgcaa 5220
   acgccggagg agcggcagga ggccgcgctg gcccgagcac gggcggaagg ccgcgtgccc 5280
   ctggtccgtc gtcgccgccg gacccgttgg tgatgcttgc ggcgctggcc tgggtggtac 5340
   cagggggctc gcgccccctg gaccccccgg cccagcggca cgacccgcgc cggacgttcc 5400
   agcggtgccg gttccgttgg cgcgggccgc accgctggag tggtgccgag ggtccggtga 5460
   gaaggtggtc accagcccgt cgaccagcag gagcgcgtcc gcatgaccag ccccgatgac 5520
   ctggacgact acctgcggga actactgcgc agggagaagg ccggcgagcg gcaccggatc 5580
   gaggttggcc cgtacacggc gatgctggtc acgggggcga tccagtgggc gatccggcgc 5640
   ggtgagctgg ccgagcacgg gccggtgatg ttcgagcggc tgctggacca gctccggctg 5700
   ctgttcgccg acgacccgag gggcgcggag ctgatctata tctatgatct cgcagtctcc 5760
   ggcgagcacc ggaggcaggg cattgccacc gtccaacgcg cttggtgctt atgtgatcta 5820
   cgtgcaagca gattacggtg acgatcccgc agtggctctc tatacaaagt tgggcatacg 5880
   ggaagaagtg atgcactttg atcagcccga cccgagcacg cgccggcacg cctggtcgat 5940
   gtcggaccgg agttcgaggt acgcggcttg caggtccagg aaggggacgt ccatgcgagt 6000
   gtccgttcga gtggcggctt gcgcccgatg ctagtcgcgg ttgatcggcg atcgcaggtg 6060
   cacgcggtcg atcttgacgg ctggcgagag gtgcggggag gatctgaccg acgcggtcca 6120
   cacgtggcac cgcgatgctg ttgtgggctg gacaatcgtg ccggttggta ggatcatcgt 6180
   ccattccgac agcatcgcca gtcactatgg cgtgctgcta gcgctatatg cgttgatgca 6240
   atttctatgc ctctaaggag gtgacaactg ctggccgcgc tcggcgggct ggtcgccgcc 6300
   gtcctgaacg gcgcgccggc catctgacct tgacgccgtc ggcccgctct tgctatccct 6360
   atatcagaac tactgatata gggagcgatg catgagcaca gcggtcggca acgggcgggt 6420
   ccggacggag ccgtggggcg agacggttct ggtggagttc gacgaaggca tcgcctgggt 6480
   catgctcaac cggccggaca agcgcaacgc catgaacccc accctgaacg acgagatggt 6540
   gcgggtgctg gaccacctgg agggcgacga ccgctgccga gtgctggtgc tgaccggcgc 6600
   gggcgagtcg ttctccgcgg gcatggacct caaggagtac ttccgcgagg tcgacgccac 6660
   cggcagcacc gccgtgcaga tcaaggtgcg gcgggccagc gcggagtggc agtggaagcg 6720
   gctggcgaac tggagcaagc cgacgatcgc gatggtcaac ggctggtgct tcggcggcgc 6780
   gttcaccccg ctggtggcct gcgacctggc cttcgccgac gaggacgcgc ggttcgggct 6840
   gtccgaggtc aactggggca tcccgccggg cggcgtggtc agccgggcgc tggcggcgac 6900
   cgtgccgcag cgcgacgcgc tgtactacat catgaccggt gagcccttcg acggcccgcc 6960
   gcgcgcggag atgcgcctgg tcaacgaggc gctgcccgcc gaccggctgc gggagcgcac 7020
   ccgcgaggtg gcgctgaagc tcgcgtcgat gaaccaggtg gtcctgcacg cggccaagac 7080
   cgggtacaag atcgcccagg agatgccctg ggagcaggcc gaggactacc tctacgccaa 7140
   gctcgaccag tcccagttcg ccgacaaggc gggcgcccgc gccaaggggc tgacccagtt 7200
   cctcgaccag aagtcctacc ggcccggcct gagcgccttc gacccggaga agtagtgcgc 7260
   aaccagggtc tgggctcctg gccggtgcgc cgcgccagga tgagcccgca cgcgacagcc 7320
   gtccggcacg gcgggacggc gctgacctac gccgagctgt cccgccgcgt cgcgcggctc 7380
   gccaacgggc tgcgggccgc cggggtccgc cccggcgacc gggtggccta cctcgggccg 7440
   aaccacccgg cctacctgga gaccctgttc gcgtgcgggc aggccggcgc ggtgttcgtg 7500
   ccgctgaact tccggctggg cgtcccggaa ctggaccacg cgctggccga ctccggcgcg 7560
   tcggtcctta tccacacccc ggagcacgcg gagacggtcg cggcgctcgc cgccggccgg 7620
   ctgctgcgcg tgcccgcggg cgagctggac gccgcggacg acgagccgcc cgacctgccc 7680
   gtcggcctcg acgacgtgtg cctgctgatg tacacctcgg gcagcaccgg acgccccaag 7740
   ggcgcgatgc tcacccacgg caacctcacc tggaactgcg tcaacgtcct ggtggagacc 7800
   gacctggcga gcgacgagcg ggcactggtc gccgcgccgc tgttccacgc cgccgcgctc 7860
   ggcatggtgt gcctgcccac cctgctcaag ggcggcacgg tgatcctgca ctccgcgttc 7920
   gaccccggcg ccgtgctgtc cgcggtggaa caggagcggg tcacgctcgt gttcggcgtg 7980
   cccacgatgt accaggcgat cgccgcgcac ccgcggtggc gcagcgccga cctgtccagc 8040
   ctgcggaccc tgctgtgcgg cggcgcgccg gtgcccgccg acctcgccag ccgctacctc 8100
   gaccgcgggc tcgcgttcgt gcagggctac ggcatgaccg aggccgcgcc gggcgtgctg 8160
   gtcctcgacc gcgcgcacgt cgcggagaag atcggctccg ccggggtgcc ctcgttcttc 8220
   accgacgtgc ggctggccgg cccgtccggc gagccggtgc cgccggggga gaagggcgag 8280
   atcgtggtca gcgggcccaa cgtgatgaag ggctactggg gcaggccgga ggcgaccgcc 8340
   gaggtgctgc gcgacgggtg gttccactcc ggcgacgtgg ccaccgtgga cggcgacggg 8400
   tacttccacg tcgtcgaccg gctcaaggac atgatcatct ccggcggcga gaacatctac 8460
   ccggccgagg tggagaacga gctgtacggc tacccgggtg tggaggcgtg cgccgtgatc      8520
   ggcgtgccgg acccgcgctg gggcgaggtg ggcaaggcgg tcgtcgtgcc cgccgacggg      8580
   agccgcatcg acggcgacga gctgctggcc tggctgcgca cccggctggc cgggtacaag      8640
   gtgcccaagt cggtcgagtt caccgaccgg ctgcccacga ccggctccgg caagatcctc      8700
   aagggcgagg tccgccgccg cttcggctga ccaggggccg atgaaccccg ctcatgcggc      8760
   cctgccggcc cgctgcggct actctgtggt ctagaactag tggatccccc gggctgcagg      8820
   aattcgatat caagcttcac gctgccgcaa gcactcaggg cgcaagggct gctaaaggaa      8880
   tccgcagaaa cggtgctgac cccggatgaa tgt                                   8913
<210> 6
   <211> 10254
   <212> DNA
   <213> Amycolatopsis
<220>
   <223> >p6sui?vdh::permE*::echfcs
<400> 6
   cagctactgg gctatctgga caagggaaaa cgcaagcgca aagagaaagc aggtagcttg      60
   cagtgggctt acatggcgat agctagactg ggcggtttta tggacagcaa gcgaaccgga     120
   attgccagct ggggcgccct ctggtaaggt tgggaagccc tgcaaagtaa actggatggc     180
   tttcttgccg ccaaggatct gatggcgcag gggatcaaga tccccgtagc ggggatccgg     240
   ggaattcccc aatgtcaagc acttccggaa tcgggagcgc ggccgatgca aagtgccgat     300
   aaacataacg atctttgtag aaaccatcgg cgcagctatt tacccgcagg acatatccac     360
   gccctcctac atcgaagctg aaagcacgag attcttcgcc ctccgagagc tgcatcaggt     420
   cggagacgct gtcgaacttt tcgatcagaa acttctcgac agacgtcgcg gtgagttcag     480
   gctttttcat atctcattgc ccccggacga gcgtctgctc cgccattcgc cgtccgccgt     540
   gccaatcgga tcagccgtcc aaatgcggga ttttcgttag tcggaggcca aacggcattg     600
   agcgtcagca tatcatcagc gagctgaaga aagacaatcc ccgatccgct ccacgtgttg     660
   ccccagcaat cagcgcgacc ttgcccctcc aacgtcatct cgttctccgc tcatgagctc     720
   agccaatcga ctggcgagcg gcatcgcatt cttcgcatcc cgcctctggc ggatgcagga     780
   agatcaacgg atctcggccc agttgaccca gggctgtcgc cacaatgtcg cgggagcgga     840
   tcaaccgagc aaaggcatga ccgactggac cttccttctg aaggctcttc tccttgagcc     900
   acctgtccgc caaggcaaag cgctcacagc agtggtcatt ctcgagataa tcgacgcgta     960
   ccaacttgcc atcctgaaga atggtgcagt gtctcggcac cccataggga acctttgcca    1020
   tcaactcggc aagatgcagc gtcgtgttgg catcgtgtcc cacgccgagg agaagtacct    1080
   gcccatcgag ttcatggaca cgggcgaccg ggcttgcagg cgagtgaggt ggcaggggca    1140
   atggatcaga gatgatctgc tctgcctgtg gccccgctgc cgcaaaggca aatggatggg 1200
   cgctgcgctt tacatttggc aggcgccaga atgtgtcaga gacaactcca aggtccggtg 1260
   taacgggcga cgtggcagga tcgaacggct cgtcgtccag acctgaccac gagggcatga 1320
   cgagcgtccc tcccggaccc agcgcagcac gcagggcctc gatcagtcca agtggcccat 1380
   cttcgagggg ccggacgcta cggaaggagc tgtggaccag cagcacaccg ccgggggtaa 1440
   ccccaaggtt gagaagctga ccgatgagct cggcttttcg ccattcgtat tgcacgacat 1500
   tgcactccac cgctgatgac atcagtcgat catagcacga tcaacggcac tgttgcaaat 1560
   agtcggtggt gataaactta tcatcccctt ttgctgatgg agctgcacat gaaccgagcg 1620
   ggctcgatcc cctcgcgagt tggttcagct gctgcctgag gctggacgac ctcgcggagt 1680
   tctaccggca gtgcaaatcc gtcggcatcc aggaaaccag cagcggctat ccgcgcatcc 1740
   atgcccccga actgcaggag tggggaggca cgatggccgc tttggtcgat actggcttaa 1800
   ctatgcggca tcagagcaga ttgtactgag agtgcaccat atgcggtgtg aaataccgca 1860
   cagatgcgta aggagaaaat accgcatcag gcgctcttcc gcttcctcgc tcactgactc 1920
   gctgcgctcg gtcgttcggc tgcggcgagc ggtatcagct cactcaaagg cggtaatacg 1980
   gttatccaca gaatcagggg ataacgcagg aaagaacatg tgagcaaaag gccagcaaaa 2040
   ggccaggaac cgtaaaaagg ccgcgttgct ggcgtttttc cataggctcc gcccccctga 2100
   cgagcatcac aaaaatcgac gctcaagtca gaggtggcga aacccgacag gactataaag 2160
   ataccaggcg tttccccctg gaagctccct cgtgcgctct cctgttccga ccctgccgct 2220
   taccggatac ctgtccgcct ttctcccttc gggaagcgtg gcgctttctc atagctcacg 2280
   ctgtaggtat ctcagttcgg tgtaggtcgt tcgctccaag ctgggctgtg tgcacgaacc 2340
   ccccgttcag cccgaccgct gcgccttatc cggtaactat cgtcttgagt ccaacccggt 2400
   aagacacgac ttatcgccac tggcagcagc cactggtaac aggattagca gagcgaggta 2460
   tgtaggcggt gctacagagt tcttgaagtg gtggcctaac tacggctaca ctagaaggac 2520
   agtatttggt atctgcgctc tgctgaagcc agttaccttc ggaaaaagag ttggtagctc 2580
   ttgatccggc aaacaaacca ccgctggtag cggtggtttt tttgtttgca agcagcagat 2640
   tacgcgcaga aaaaaaggat ctcaagaaga tcctttgatc ttttctacgg ggtctgacgc 2700
   tcagtggaac gaaaactcac gttaagggat tttggtcatg agattatcaa aaaggatctt 2760
   cacctagcca cccgatgagc cgccgccgcg ccgtccagcg aggagcccgc cgggccgagc 2820
   ccgtcgaccc gcacgcgtcc atcgagggcc agctggccct gttcggcgac gacccgcagc 2880
   ccgagcccgt caaggccgag ctggccgccg tcgacccggc ggaggcgtcc cgatgaaccc 2940
   gcgggcgagc ggcaccggat cgaggttggc ccgtacacgg cgatgctggt cacgggggcg 3000
   atccagtggg cgatccggcg cggtgagctg gccgagcacg ggccggtgat gttcgagcgg 3060
   ctgctggacc agctccggct gctgttcgcc gacgacccga ggggcgcgga gctgatctat 3120
   atctatgatc tcgcagtctc cggcgagcac cggaggcagg gcattgccac cgtccaacgc 3180
   gcttggtgct tatgtgatct acgtgcaagc agattacggt gacgatcccg cagtggctct 3240
   ctatacaaag ttgggcatac gggaagaagt gatgcacttt gatgatcttc cggatggctc 3300
   gagtttttca gcaagattcg tgtgggtgtt cgggtacggg ttcgtggcgc ggatgcggcc 3360
   gggcgaacgc acgccgtggt cgtacttcgc ggtgatctcg tgggccggga tgcgcggcgt 3420
   ggtcacgctc gccgccgcgt tcgtgctccc ggacgacacg ccgcagcgcg cggtgctggt 3480
   gctggcggcg ttcgtcgtcg tggccggaac gctgctgctg cacggcatga ccctgcccgg 3540
   cctggtgcgc cgcctgcgcc tgccgccgcc caacccggcg gaggacgcgc tgcaggaagc 3600
   cgcgctgctg cacgacatga cgtccgcggg cctggcccgg ctcgaggaga tccgccgccc 3660
   ggccgacccg cccgaggtca tcgaccggtt gcgggacaag cagtcctacc ggtcggactc 3720
   ggcgtgggag aacctcggca ggctcagcgc gctggaggag acccccagcg acgcgtaccg 3780
   tcggctgcgc gccgagatgc tcgcggccga gcgggacgtc ctgctcaccg cgcgggaccg 3840
   gggcaccgcc gacgacgagg tgctccggcg ggtgatggac ggactggaca tcgaggagtc 3900
   gctgctggag cgacccgagg aggagccggg catggaacgt gagctggaga cccccgaggc 3960
   cacggcgggc acctgcgagc acctggagaa ggcacgcgtg ccggagaccg acgccgaggg 4020
   ctgcgtcgcg agcaccggag gcagggcatt gccaccgtcc aacgcgcttg gtgcttatgt 4080
   gatctacgtg caagcagatt acggtgacga tcccgcagtg gctctctata caaagttggg 4140
   catacgggaa gaagtgatgc actttgatca gcccgacccg agcacgcgcc ggcacgcctg 4200
   gtcgatgtcg gaccggagtt cgaggtacgc ggcttgcagg tccaggaagg ggacgtccat 4260
   gcgagtgtcc gttcgagtgg cggcttgcgc ccgatgctag tcgcggttga tcggcgatcg 4320
   caggtgcacg cggtcgatct tgacggctgg cgagaggtgc ggggaggatc tgaccgacgc 4380
   ggtccacacg tggcaccgcg atgctgttgt gggctggaca atcgtgccgg ttggtaggat 4440
   catcgtccat tccgacagca tcgccagtca ctatggcgtg ctgctagcgc tatatgcgtt 4500
   gatgcaattt ctatgcctct aaggaggtga caactgctgg ccgcgctcgg cgggctggtc 4560
   gccgccgtcc tgaacggcgc gccggccatc tgaccttgac gccgtcggcc cgctcttgct 4620
   atccctatat cagaactact gatataggga gcgatgcatg agcacagcgg tcggcaacgg 4680
   gcgggtccgg acggagccgt ggggcgagac ggttctggtg gagttcgacg aaggcatcgc 4740
   ctgggtcatg ctcaaccggc cggacaagcg caacgccatg aaccccaccc tgaacgacga 4800
   gatggtgcgg gtgctggacc acctggaggg cgacgaccgc tgccgagtgc tggtgctgac 4860
   cggcgcgggc gagtcgttct ccgcgggcat ggacctcaag gagtacttcc gcgaggtcga 4920
   cgccaccggc agcaccgccg tgcagatcaa ggtgcggcgg gccagcgcgg agtggcagtg 4980
   gaagcggctg gcgaactgga gcaagccgac gatcgcgatg gtcaacggct ggtgcttcgg 5040
   cggcgcgttc accccgctgg tggcctgcga cctggccttc gccgacgagg acgcgcggtt 5100
   cgggctgtcc gaggtcaact ggggcatccc gccgggcggc gtggtcagcc gggcgctggc 5160
   ggcgaccgtg ccgcagcgcg acgcgctgta ctacatcatg accggtgagc ccttcgacgg 5220
   cccgccgcgc gcggagatgc gcctggtcaa cgaggcgctg cccgccgacc ggctgcggga 5280
   gcgcacccgc gaggtggcgc tgaagctcgc gtcgatgaac caggtggtcc tgcacgcggc 5340
   caagaccggg tacaagatcg cccaggagat gccctgggag caggccgagg actacctcta 5400
   cgccaagctc gaccagtccc agttcgccga caaggcgggc gcccgcgcca aggggctgac 5460
   ccagttcctc gaccagaagt cctaccggcc cggcctgagc gccttcgacc cggagaagta 5520
   gtgcgcaacc agggtctggg ctcctggccg gtgcgccgcg ccaggatgag cccgcacgcg 5580
   acagccgtcc ggcacggcgg gacggcgctg acctacgccg agctgtcccg ccgcgtcgcg 5640
   cggctcgcca acgggctgcg ggccgccggg gtccgccccg gcgaccgggt ggcctacctc 5700
   gggccgaacc acccggccta cctggagacc ctgttcgcgt gcgggcaggc cggcgcggtg 5760
   ttcgtgccgc tgaacttccg gctgggcgtc ccggaactgg accacgcgct ggccgactcc 5820
   ggcgcgtcgg tccttatcca caccccggag cacgcggaga cggtcgcggc gctcgccgcc 5880
   ggccggctgc tgcgcgtgcc cgcgggcgag ctggacgccg cggacgacga gccgcccgac 5940
   ctgcccgtcg gcctcgacga cgtgtgcctg ctgatgtaca cctcgggcag caccggacgc 6000
   cccaagggcg cgatgctcac ccacggcaac ctcacctgga actgcgtcaa cgtcctggtg 6060
   gagaccgacc tggcgagcga cgagcgggca ctggtcgccg cgccgctgtt ccacgccgcc 6120
   gcgctcggca tggtgtgcct gcccaccctg ctcaagggcg gcacggtgat cctgcactcc 6180
   gcgttcgacc ccggcgccgt gctgtccgcg gtggaacagg agcgggtcac gctcgtgttc 6240
   ggcgtgccca cgatgtacca ggcgatcgcc gcgcacccgc ggtggcgcag cgccgacctg 6300
   tccagcctgc ggaccctgct gtgcggcggc gcgccggtgc ccgccgacct cgccagccgc 6360
   tacctcgacc gcgggctcgc gttcgtgcag ggctacggca tgaccgaggc cgcgccgggc 6420
   gtgctggtcc tcgaccgcgc gcacgtcgcg gagaagatcg gctccgccgg ggtgccctcg 6480
   ttcttcaccg acgtgcggct ggccggcccg tccggcgagc cggtgccgcc gggggagaag 6540
   ggcgagatcg tggtcagcgg gcccaacgtg atgaagggct actggggcag gccggaggcg 6600
   accgccgagg tgctgcgcga cgggtggttc cactccggcg acgtggccac cgtggacggc 6660
   gacgggtact tccacgtcgt cgaccggctc aaggacatga tcatctccgg cggcgagaac 6720
   atctacccgg ccgaggtgga gaacgagctg tacggctacc cgggtgtgga ggcgtgcgcc 6780
   gtgatcggcg tgccggaccc gcgctggggc gaggtgggca aggcggtcgt cgtgcccgcc 6840
   gacgggagcc gcatcgacgg cgacgagctg ctggcctggc tgcgcacccg gctggccggg 6900
   tacaaggtgc ccaagtcggt cgagttcacc gaccggctgc ccacgaccgg ctccggcaag 6960
   atcctcaagg gcgaggtccg ccgccgcttc ggctgaccag gggccgatga accccgctca 7020
   tgcggccctg ccggcccgct gcggctactc tgtggtctag aactagtgga tcccccgggc 7080
   tgcaggaatt cgatatcaag cttcacgctg ccgcaagcac tcagggcgca agggctgcta 7140
   aaggaatccg cagaaacggt gctgaccccg gatgaatgga ctgcctgcgc gagggctcgt 7200
   cgtgggtgca cctgcggctg tgcctggagt gcgggcacgt cggctgctgc gactcgtcgc 7260
   cgcgcaagca cgccacgcag cacttccacg acacgcgtca cccggtgatc cgcagctacg 7320
   agcccggcga gaactggcgc tggtgcttcg tggacgggat cgtcggctga gcgggggcgc 7380
   cgtcggcgcc cccgccgcgc gtgtcactcc tgatgcgcga ggttttccgc catcctcctg 7440
   ggctgttcgg gccgcgccag tgggtctttc actgagtgga aggccggaaa actgtcggtg 7500
   gtggctgcga tgatcgcgct cgtggacgac tcgaagatcc ggcggacgct cgcctcgcac 7560
   ggcccggtgc ggcggcggcc acgggcggtg ctcgaacgga tgctggcgct ggcgggggag 7620
   gacctgtcgc ccgcggagcc ggtggcggca ctggaggcgc ggatcgcgtc gctgctgggc 7680
   aagccggcgg cgttgttctt cccgagcggg acgatggcgc agcaggcggc gctgcgggtg 7740
   cacgcggacc ggtccgggcg gcggacgttc gccgcgcacc cgtacacgca tctggacaac 7800
   tgggaggaca aggggtacgc ggcggtgcac gggctgcggt tccggccggt gggggaccgc 7860
   aacgagctga tgacgccggc ggacctggag gcggtggggg agccgctcgc ggccgtggtg 7920
   tgggagctgc cgcagcggga cctcggcggg ttgctaccgt cgtgggacga gctggtcgcg 7980
   cagaccgggc tcgcgcgctc ccgcggcgcg gcggcgcacc tggacggcgc tcggctgtgg 8040
   gagtcgcaac cgttctacgg caaggatttc gccgagatcg ccggcctgtt cgacagcgtg 8100
   tacgtgtcgc tctacaaagg actgcaaatc tttctagaaa tcgtccattc cgacagcatc 8160
   gccagtcact atggcgtgct gctagaggat ccattggtac cattaccctg ttatccctag 8220
   aacgtccggg cttgcacctc acgtcacgtg aggaggcagc gtggacggcg tggtaccaag 8280
   cttattggca ctagtcgagc aacggaggta cggacatgct gcggcccgtc gaaaccccga 8340
   cccgcgagat caagaagctg gacggcctgt gggcgttcag cctcgaccgg gagaactgcg 8400
   gtatcgacca gcggtggtgg gagagcgccc tccaggagtc gcgcgccatc gccgtccccg 8460
   gcagcttcaa cgaccagttc gcggacgcgg acatccgcaa ctacgcgggg aacgtctggt 8520
   atcagcggga ggtgttcatc cccaagggct gggccggtca gcgcatcgtg ctgcgcttcg 8580
   acgccgtgac ccactacggc aaggtctggg tcaacaacca ggaagtgatg gagcaccagg 8640
   gcggctacac gcccttcgag gcggacgtga ccccgtacgt catcgccggc aagtcggtcc 8700
   gcatcacggt ctgcgtcaac aacgagctca actggcagac catcccgccc ggcatggtga      8760
   tcacggacga gaacggcaag aagaagcaga gctacttcca cgacttcttc aactacgccg      8820
   gcatccaccg ctcggtcatg ctgtacacga cgccgaacac ctgggtcgac gacatcacgg      8880
   tggtcaccca cgtggcccag gactgcaacc acgcgtccgt ggactggcag gtcgtcgcca      8940
   acggcgacgt gagcgtggag ctccgcgacg ccgagcagca ggtcgtggcg accgggcagg      9000
   gcacctcggg gaccctccag gtcgtgaacc cccacctctg gcagccgggt gagggctacc      9060
   tgtacgagct gtgcgtgacg gcgaagagcc agaccgagtg cgacatctac cccctgcgcg      9120
   tcggcatccg gtccgtggcg gtcaagggcg agcagttcct gatcaaccac aagccgttct      9180
   acttcacggg cttcggtcgg cacgaggacg ccgacctccg cggcaagggc ttcgacaacg      9240
   tcctgatggt ccacgaccac gccctgatgg actggatcgg cgccaactcg taccggacct      9300
   cgcactaccc gtacgcggaa gagatgctgg actgggcgga cgagcacggg atcgtcgtca      9360
   tcgacgaaac cgccgcggtg ggcttcaacc tgtccctcgg catcggcttc gaggccggca      9420
   acaagccgaa agagctctac tccgaagagg ccgtcaacgg cgaaacccag caggcccacc      9480
   tccaggcgat caaagagctc atcgcccgcg acaagaacca cccgtccgtc gtgatgtggt      9540
   cgatcgccaa cgagccggac acccggccgc aggtgcccgg ggagtacttc gccccgctgg     9600
   ccgaggccac ccggaagctc gaccccaccc ggcccatcac ctgcgtgaac gtgatgttct      9660
   gcgacgccca cacggacacc atctccgacc tcttcgacgt cctgtgcctg aaccgctact      9720
   acggctggta cgtgcagtcc ggggacctgg aaacggccga gaaggtcctg gagaaagagc      9780
   tgctggcgtg gcaggagaag ctgcaccagc cgatcatcat caccgagtac ggcgtggaca      9840
   cgctggccgg tctgcactcc atgtacaccg acatgtggtc cgaagagtac cagtgcgcgt      9900
   ggctggacat gtaccaccgc gtgttcgacc gcgtcagcgc ggtcgtcggc gagcaggtgt      9960
   ggaacttcgc cgacttcgcc acctcccagg gcatcctccg cgtcgggggc aacaagaagg     10020
   gcatcttcac ccgggaccgc aagcccaagt cggcggcgtt cctgctccag aagcggtgga     10080
   ccgggatgaa cttcggggag aagccgcagc agggcgggaa gcagtgataa gcattaccct     10140
   gttatcccta ggatccgcgg ccgcgcgcga taaagcttca cgctgccgca agcactcagg     10200
   gcgcaagggc tgctaaagga atccgcagaa acggtgctga ccccggatga atgt            10254